# EUROPEAN PATENT SPECIFICATION

(11) **EP 1 297 013 B1**
(45) Date of publication and mention of the grant of the patent: **16.08.2006**
(21) Application number: 01927474.5
(22) Date of filing: 27.04.2001
(51) Int. Cl.: C07K 14/705

(54) **USE OF TACI AS AN ANTI-TUMOR AGENT**
VERWENDUNG VON TACI ALS ANTITUMORMITTEL
TACI UTILISEE EN TANT QU'AGENT ANTITUMORAL

(30) Priority: 27.04.2000 US 199946 P
(43) Date of publication of application: 02.04.2003
(73) Proprietor: Biogen Idec MA Inc., Cambridge, Massachusetts 02142 (US); Apoxis SA, 1066 Epalinges (CH)
(72) Inventor: AMBROSE, Christine, Reading, MA 01867 (US); THOMPSON, Jeffrey, Stoneham, MA 02180 (US); SCHNEIDER, Pascal, Ch-1066 Epalinges (CH); RENNERT, Paul, Millis, MA 02054 (US)
(74) Representative: Pohlman, Sandra M.
(86) International application number: PCT/US2001/040626
(87) International publication number: WO 2001/081417

(56) References cited:
- WO-A-00/40716
- WO-A-00/58362
- WO-A-98/39361
- CUBILLOS SUZANA ET AL: "Effect of blocking TNF on IL-6 levels and metastasis in a B16-BL6 melanoma/mouse model." ANTICANCER RESEARCH, vol. 17, no. 3C, 1997, pages 2207-2211, XP001051935 ISSN: 0250-7005
- BROWNING J L ET AL: "SIGNALING THROUGH THE LYMPHOTOXIN BETA RECEPTOR INDUCES THE DEATH OF SOME ADENOCARCINOMA TUMOR LINES" JOURNAL OF EXPERIMENTAL MEDICINE, TOKYO, JP, vol. 183, no. 3, 1 March 1996 (1996-03-01), pages 867-878, XP000571445 ISSN: 0022-1007
- RENNERT P ET AL: "A SOLUBLE FORM OF B CELL MATURATION ANTIGEN, A RECEPTOR FOR THE TUMOR NECROSIS FACTOR FAMILY MEMBER APRIL, INHIBITS TUMOR CELL GROWTH" JOURNAL OF EXPERIMENTAL MEDICINE, TOKYO, JP, vol. 192, no. 11, 4 December 2000 (2000-12-04), pages 1677-1683, XP000982115 ISSN: 0022-1007

## Description

### FIELD OF THE INVENTION

The present invention relates generally to methods of treating a mammal for a condition associated with undesired cell proliferation, including cancer.

### BACKGROUND OF THE INVENTION

Members of the tumor-necrosis factor (TNF) family of cytokines are involved in an ever-expanding array of critical biological functions. Each member of the TNF family acts by binding to one or more members of a parallel family of receptor proteins, namely, the TNF receptor family of proteins. TNF receptors, which in turn, signal intracellularly to induce a wide range of physiological or developmental responses. Many of the TNF receptor signals influence cell fate, and often trigger terminal cellular differentiation. Examples of cellular differentiation include proliferation, maturation, migration, and death.

In U.S. patent number 5,969,102, TACI (Transmembrane Activator CAML Interactor protein), a novel TNF family member receptor protein, is described. The corresponding TNF family member ligand to TACI, however, was not known.

The present invention discloses that the TNF family member APRIL, which is described in WO 99/12965, is a ligand to TACI. It is also a discovery of the present invention that TACI reagents are particularly useful in treating a mammal for a condition associated with undesired cell proliferation, including for example, cancer.

### SUMMARY OF THE INVENTION

The present invention is directed to use of a TACI reagent in the manufacture of a medicament for the prevention or treatment of a solid tumor, wherein said medicament extends the mean survival time and/or reduces solid tumor size, wherein said TACI reagent can augment or diminish ligand binding to TACI and wherein said TACI reagent is a fusion polypeptide comprising at least two segments, wherein a first segment comprises a TACI polypeptide or fragment thereof selected from the group consisting of:
(a) amino acid residues 1-166 of SEQ ID NO:1;
(b) amino acid residues 1-160 of SEQ ID NO:1;
(c) amino acid residues 1-114 of SEQ ID NO: 1;
(d) amino acid residues 32-114 of SEQ ID NO:1;
(e) a polypeptide having at least 80% amino acid sequence identity with amino acid residues 1-166 of SEQ ID NO: 1;
(f) a polypeptide having at least 80% amino acid sequence identity with amino acid residues 1-160 of SEQ ID NO: 1;
(g) a polypeptide having at least 80% amino acid sequence identity with amino acid residues 1-114 of SEQ ID NO:1; and
(h) a polypeptide having at least 80% amino acid sequence identity with amino acid residues 32-17.4 of SEQ ID NO:1, and
a second segment comprises an immunoglobulin polypeptide such solid tumors include but are not limited to cancer and specifically renal cell cancer, Kaposi's sarcoma, prostate cancer, breast cancer, sarcoma, ovarian carcinoma, rectal cancer, throat cancer, melanoma, colon cancer, bladder cancer, mastocytoma, lung cancer, mammary adenocaminoum, pharyngeal squamous cell carcinoma, gastrointestinal cancer, and stomach cancer.

It is to be understood from the foregoing general description and the following detailed description are exemplary and explanatory, and are intended to provide further explanation of the invention claimed.

### BRIEF DESCRIPTION OF THE DRAWINGS

The accompanying drawings are included to provide a further understanding of the invention, and are incorporated in, and constitute a part of this specification, illustrate several embodiments of the invention, and together with the description serve to explain the principles, of the invention.

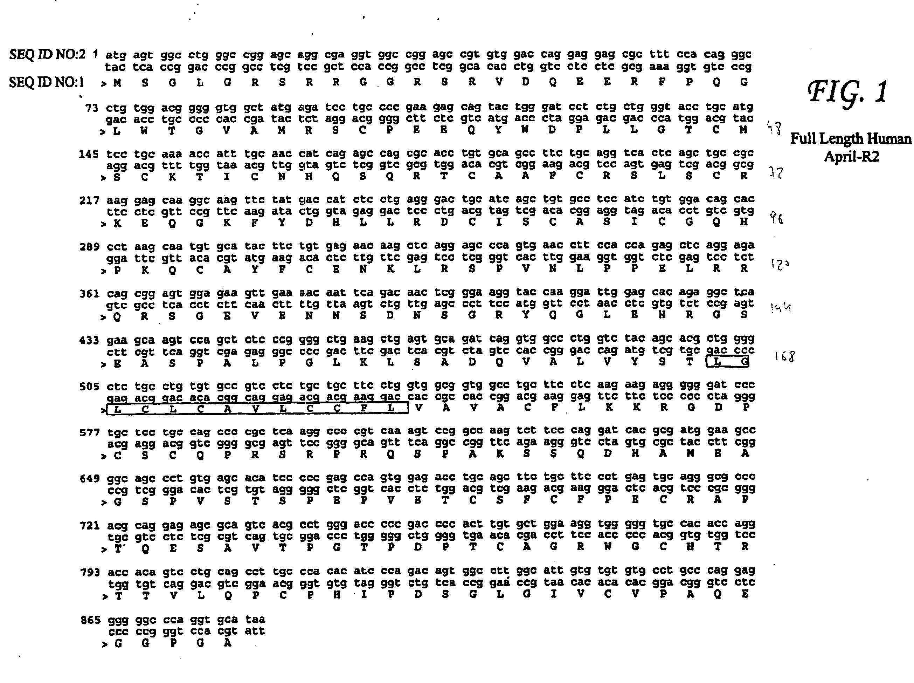

**Figure 1** is a schematic representation of the nucleic acid sequence (SEQ ID NO:2) of a cDNA for human TACI and its derived amino acid sequence (SEQ ID NO:1) as mapped in vector pCA336.

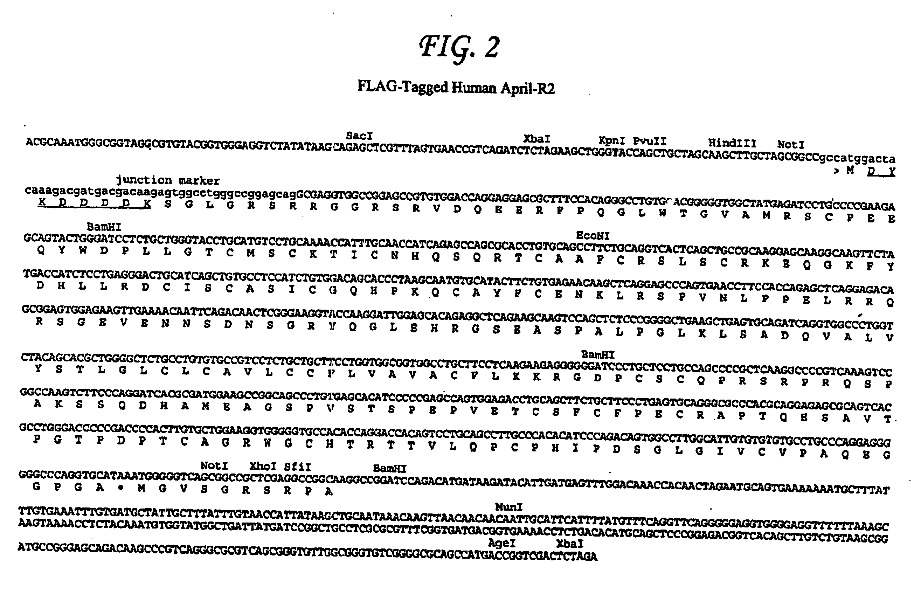

**Figure 2** is a schematic representation of the nucleic acid sequence insert in pJST552 encoding N-terminus FLAG-tagged human full length TACI, and its derived amino acid sequence.

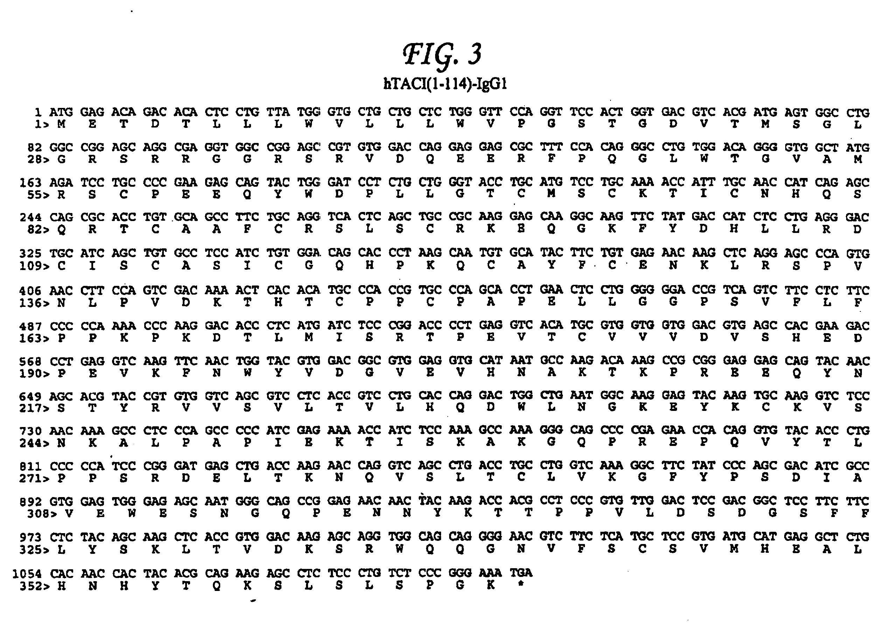

**Figure 3** is a schematic representation of the DNA sequence (SEQ ID NO:5) and its derived amino acid sequence (SEQ ID NO:6) of the TACI extracellular domain with a truncated stalk region fused to human IgG Fc. This was assembled as plasmid pJST572. The signal sequence from murine IgG-kappa, nucleotides 1-69 (amino acids 1-23), was fused in frame with the human TACI extracellular domain (amino acids 1-114 of SEQ ID NO: 1) as nucleotides 70-411 (amino acids 24-137) which was fused in frame to the hIgGl Fc as nucleotides 412-1098 (amino acids 138-366). The predicted signal peptidase cleavage site is after amino acid 20.

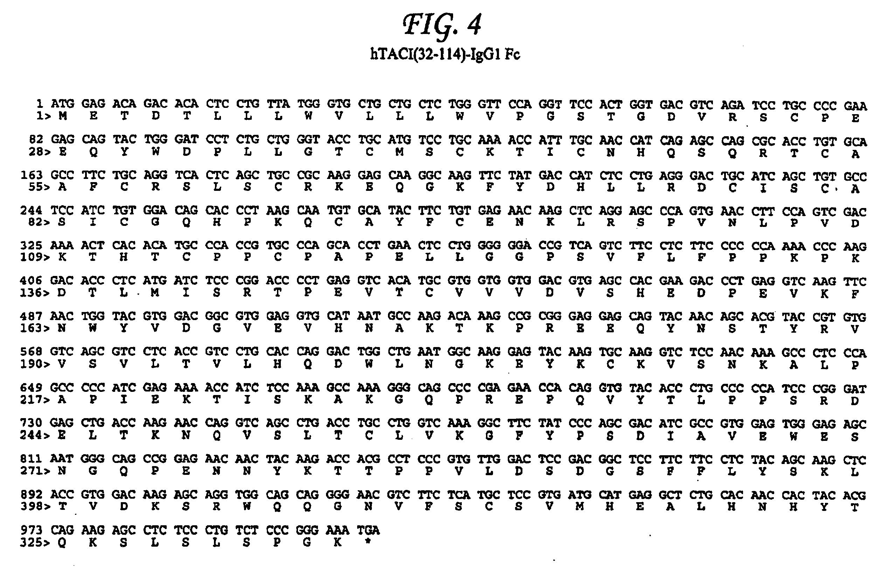

**Figure 4** is a schematic representation of the DNA sequence (SEQ ID NO:7) and its derived amino acid sequence (SEQ ID NO:8) of the TACI extracellular domain with a truncated stalk region initiating after the second methionine fused to human IgG Fc. This was assembled as plasmid pJST591. The signal sequence from murine IgG-kappa, nucleotides 1-66 (amino acids 1-22), was fused in frame with human TACI extracellular domain (amino acids 32-114 of SEQ ID NO:1) as nucleotides 67-315 (amino acids 23-105) which was fused in frame to the hIgG1 Fc as nucleotides 316-1002 (amino acids 106-334). The predicted signal peptidase cleavage site is after amino acid 20.

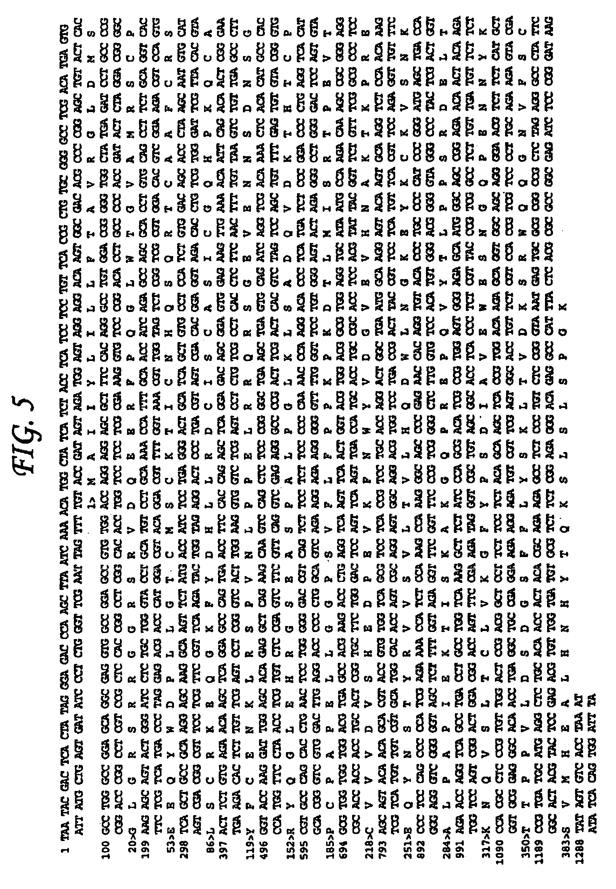

**Figure 5** is a schematic representation of the nucleic acid sequence and its derived amino acid sequence of the complete extracellular domain of TACI fused to a human IgG-Fc sequence, as assembled in plasmid PS882, wherein there is a short hemaglutinin (HA)-signal sequence in frame with the native methionine (amino acid 18) and TACI extracellular domain sequence through amino acid 177 (valine, which is amino acid 160 of TACI) after which there is a human IgG-Fc construct in frame.

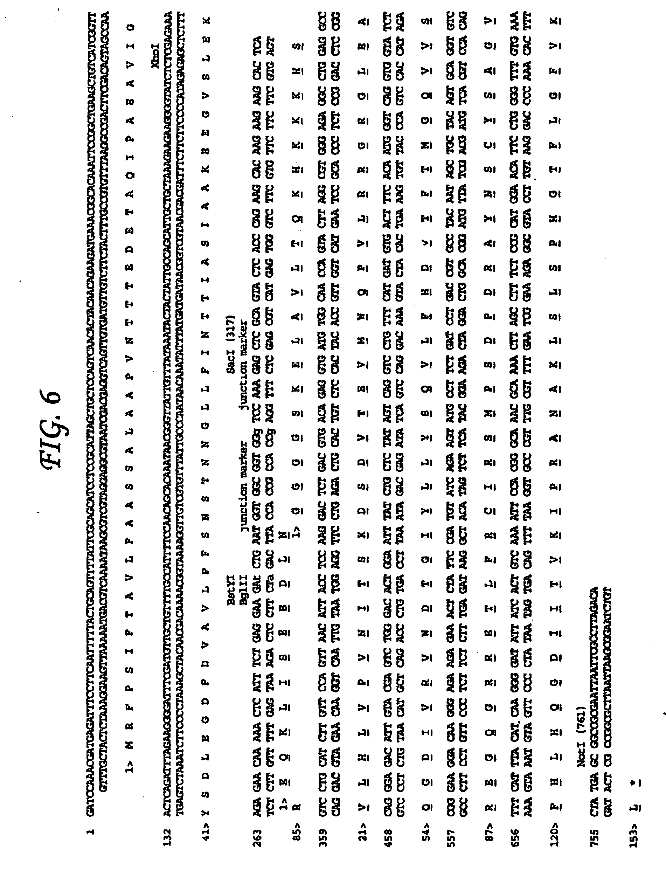

**Figure 6** is a schematic representation of the nucleic acid sequence (SEQ ID NO:4) and its derived amino acid sequence (SEQ ID NO:3) of a myc-tagged murine APRIL construct for expression in *Pichia pastoris* cells, as mapped in plasmid pCMM276, including the alpha mating factor signal sequence, which is cleaved off; the myc epitope (first 11 amino acids after the signal sequence; underlined); a short linker region (next 8 amino acids); and the soluble extracellular domain of murine APRIL coding sequence from amino acid 20, which is an alanine, to the first stop codon.

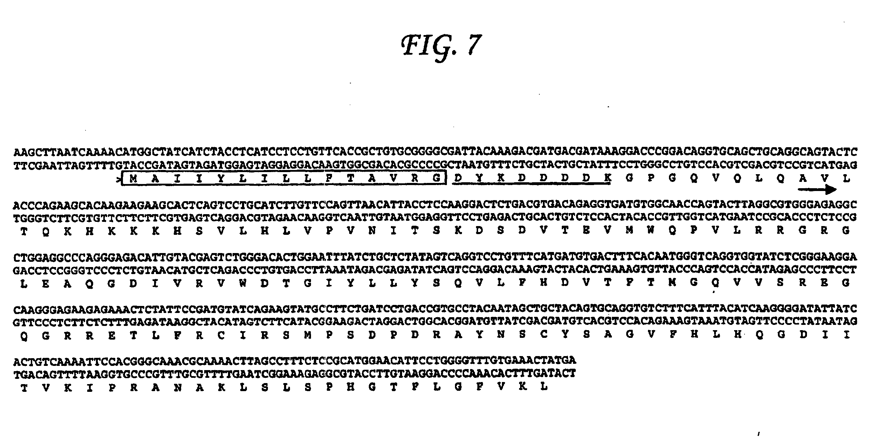

**Figure 7** is a schematic representation of the nucleic acid sequence of FLAG-tagged soluble extracellular domain of murine APRIL, and the corresponding amino acid sequence, as mapped in the mammalian expression plasmid PS784, also known as LT032, wherein there is an HA-signal sequence (boxed), the FLAG epitope (underlined), a short linker sequence, then soluble murine APRIL sequence (arrow beginning at alanine).

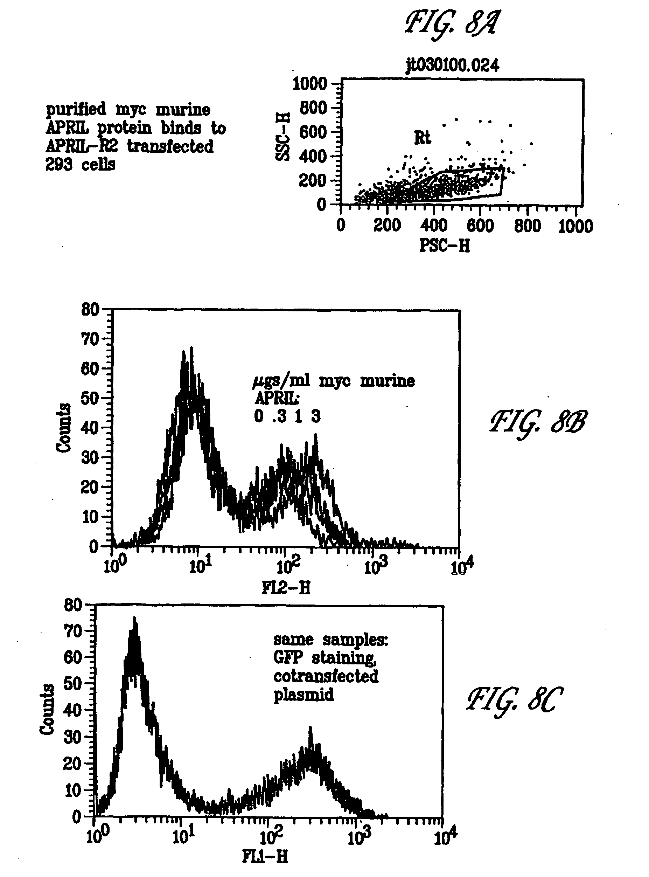

**Figure 8** is a schematic representation of purified myc-tagged murine APRIL binding to TACI transfected cells. 293EBNA cells were transfected with expression plasmid pJST552 that expressed FLAG-tagged full length human TACI. Cells were harvested 48 hours later using 5 mM EDTA and stained with myc-tagged murine APRIL at various concentrations. No protein control was stained with detection reagents (rabbit anti-murine APRIL and donkey anti-rabbit-PE (Jackson Immunoresearch)). Staining in FL1 of protein encoded by the cotransfected GFP expression plasmid illustrates expression efficiency.

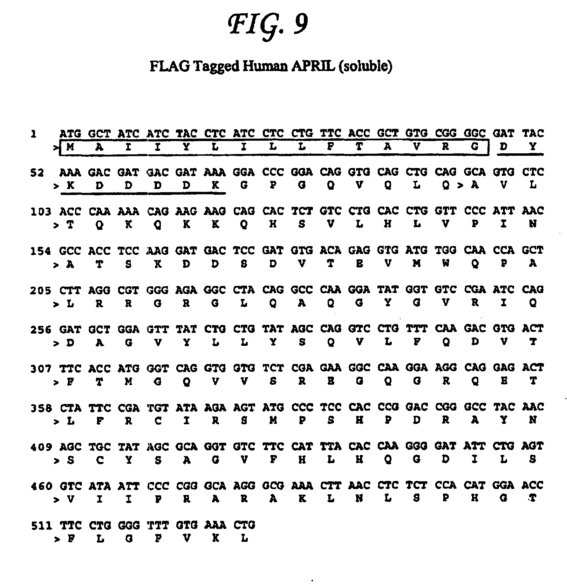

**Figure 9** is a schematic representation of the DNA sequence of FLAG tagged soluble extracellular domain of human APRIL (SEQ ID NO:9) and the corresponding amino acid sequence (SEQ ID NO:10) as cloned in the mammalian expression vector LT033. This construct contains an HA signal sequence tag (boxed), the FLAG epitope tag (underlined), and a short linker sequence fused to soluble human APRIL (arrow, beginning at alanine).

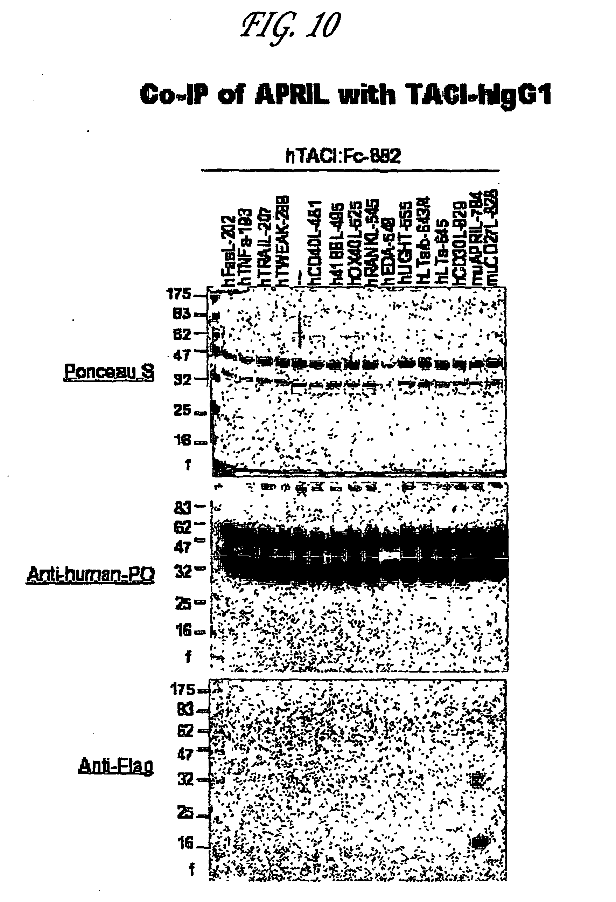

**Figure 10** is a series of representations of Western blots delineating the immunoprecipitation of FLAG-tagged murine APRIL using human TACI(1-160)-Ig (hTACI(1-160)-Ig) fusion protein. Figure 10A is a representation of a Western blot showing Ponceau-S staining of protein loads for the ligands. Figure 10B is a representation of a Western blot showing the amount of hTACI(1-160)-Ig used in the immunoprecipitations by revealing the IgG-Fc portion. Figure 10C is a representation of a Western blot showing that only APRIL immunoprecipitated with hTACI(1-160)-Ig, as evidenced by revealing the FLAG-tag.
**Figure 11** shows the results of *in vivo* tumor growth inhibition of HT29 colon adenocarcinoma cells by hTACI(1-114)-Ig.

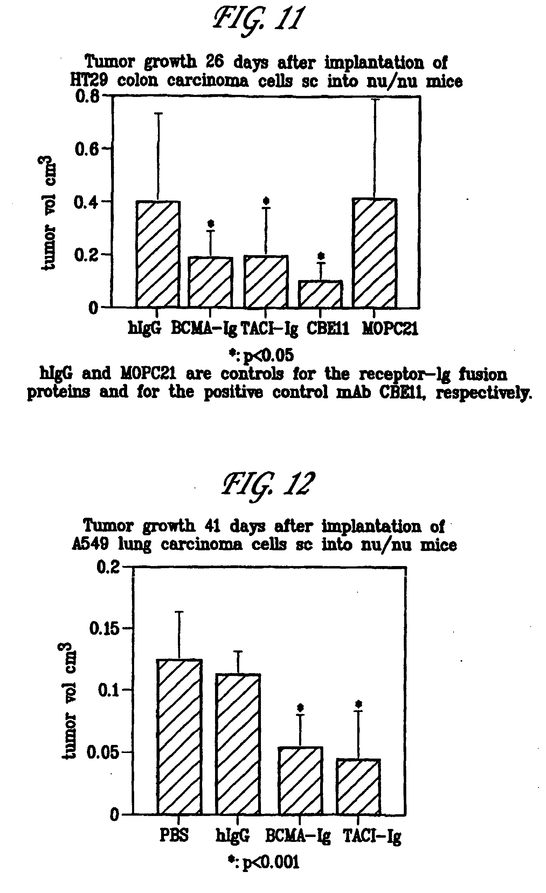

**Figure 12** shows the results of *in vivo* tumor growth inhibition of A594 lung carcinoma cells by hTACI(1-114)-Ig.

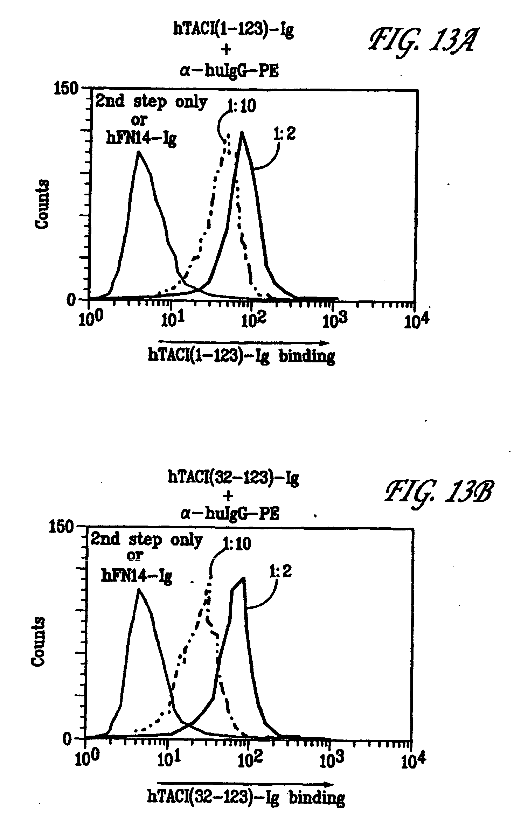

**Figure 13** shows a histogram overlay of hTACI(1-114)-Ig and hTACI(32-114)-Ig binding to cells stably expressing surface murine APRIL.

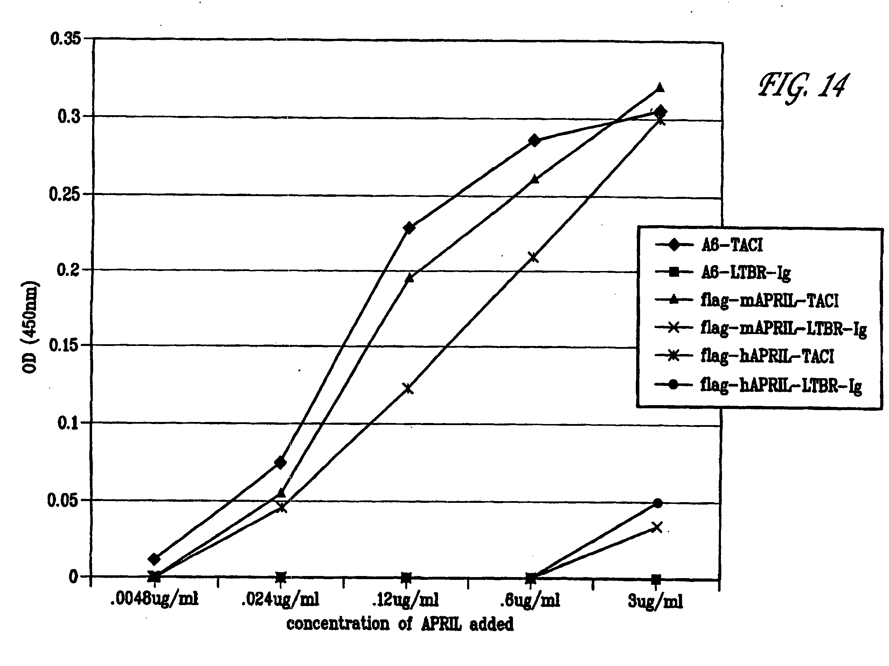

**Figure 14** shows an plot of an ELISA analysis of showing binding of murine and human APRIL to hTACI(32-114)-Ig.

### DETAILED DESCRIPTION OF THE INVENTION

It has now been unexpectedly discovered that TACI is a receptor for APRIL (A Proliferation Inducing Ligand). It is also an unexpected discovery of the present invention that TACI reagents can be used in the manufacture of a medicament to treat a mammal for a condition associated with solid tumors. Such a medicament extends mean survival time of said mammal by about 10 %, 15%, 20%, 25% or more compared to the absence of administering the medicament for said condition. Moreover, it is an unexpected discovery of the present invention that the medicaments can be used to reduce the size of a tumor located on or in a mammal, wherein said medicament reduces the size: of said tumor by about 10 %, 15%, 20%, 23% or more as compared to not administering the medicament.

As used herein, "treating or treatment" means an approach for obtaining beneficial or desired clinical results. For purposes of this invention, beneficial or desired clinical results include, but are not limited to, one or more of the following: alleviation of symptoms, diminishment of extent of disease, stabilized (*e.g.,* not worsening) state of disease, preventing spread (*e.g*., metastasis) of disease, preventing occurrence or recurrence of disease, delay or slowing of disease progression, amelioration of the disease state, and remission (whether partial or total). Also encompassed by "treatment" is a reduction of pathological consequences of a condition associated with undesired cell proliferation, including specifically, cancer.

By "mammal" as used herein means any mammal including humans, cows, horses, dogs, rats, mice and cats. In preferred embodiment of the invention, the mammal is a human.

"A condition associated with undesired cell proliferation" as used herein includes but is not limited to cancer, specifically, conditions comprising at least one solid tumor, including, but not limited to renal cell cancer, Kapoel's sarcoma, breast cancer, sarcoma, ovarian carcinoma, rectal cancer, throat cancer, melanoma, colon cancer, bladder cancer, mastocytoma, lung cancer, mammary adenocarcinoma, pharyngea! squamous cell carcinoma, gastrointestinal cancer or stomach cancer. Preferably, the cancer is naestocytoma, melanoma, lymphoma, mammary adenecarcinonia, prostate and breast cancer. Also contemplated are other conditions associated with undesired cell proliferation including but not limited to cellular hypetproliferation (hyperplasia), selected from the group consisting of for example, scleroderma, pannus formation in rheumatoid arthritis, post-surgical scairing and lung, liver, and uterine fibrosis.

As used herein "administering" means the medicament can be administered alone or in combination with other pharmaceutical agents and cam be combined with a physiotogncall.y. acceptable carrier therefor. The effective amount and method of administration of the particular medicament can vary based on the individual mammal and the stage of the disease and other factors evident to one skilled in the art. The route(s) of administration useful in a particular application are apparent to one of skill in the art.

Routes of admnistration include but are not limited to topical, transdermal, parenteral, gastrointestinal, transbronchial and transelveolar. Topical admnistration in accomplished via a topically applied cream, gel, rinse, etc. containing an oligonucleotide conjugate. Transdermal administration is accomplished by application ofa cream, rinse, gel, etc. capable of allowing the medicament to penetrate the skin and enter the blood stream. Parenteral routes of administration include but are not limited to electrical or direct injection such as direct injection into a central venous line, intravenous, intramuscular, intraperitoneal or subcutaneous injection. Gastrointestinal routes of administration include but are not limited to ingestion and rectal administration. Transbronchial and transalveolar routes of administration include but are not limited to inhalation, either via the mouth or intranssally.

An "effective amount" as used herein is an amount sufficient to effect beneficial or desired clinical results (Stites *et al.,* BASIC & CLINICAL IMMUNOLOGY, Lange Medical Publications, Los Altos, California, 1982). An effective amount can be administered in one or more administrations as described herein. For purposes of this invention, an effective amount of a medicament comprising a TACI reagent is an amount sufficient to extend mean survival time of a mammal by at least 10%, alternately 13%, 20% or 25% in comparison to mean survival in the absence of administering said medicament. Detection and measurement of indicators of efficacy are generally based on measurement of clinical symptoms associated with the disease state, such as increased average life expectancy after treatment with a medicament comprising a TACI reagent.

An effective amount of a medicament comprising a TACI reagent for reducing the size of a tumor in or on a mammal is an amount sufficient to reduce tumor size on or in a mammal by at least 10%, alternatively 15%, 20% or 25% more than in the absence of administering said medicament. Methods for measuring tumor size in a mammal are known to those of skill in the art and can be measured by non-invasive procedures, including but not limited to using a micrometer to measure the tumor diameter, if the tumor is located on the exterior surface of a mammal. Alternatively if the tumor is located in the interior of the mammal one can use MRI to measure the tumor diameter. Invasive procedures include surgically removing the tumor from the mammal and weighing the tumor and comparing the size of the tumor to pretreatment with the medicament comprising a TACI reagent.

As used herein "a TACI reagent" means those reagents that can influence how the TACI signal is interpreted within the cell including antagonistic-TACI reagents that can diminish ligand binding to TACI, including for example, TACI fusion proteins such as TACI-IgG Fc. Also contemplated are agonistic TACI reagents that can augment ligand binding to TACI, including for example, antibodies to TACI such as anti-TACI monoclonal antibodies. The term Fc domain refers to a part of the molecule comprising the hinge, CH2 and CH3 domains, but lacking the antigen binding sites. The term is meant to include the equivalent regions of an IgG, an IgM and other antibody isotypes.

As used herein, "extend mean survival time" means that the average life expectancy associated with a particular condition associated with undesired cell proliferation is on average increased. Average life expectancy is known to those of skill in the art for various forms of cancer in various forms of mammals, including various forms of cancer in humans, and various forms of cancer in rodents, including mice. Furthermore, as used herein, an extended mean survival time of, for example, about 10% or more as compared to mean survival time in the absence of administering a medicament comprising a TACI reagent, means for example, that for a human patient with a form of cancer that has an survival time of about 365 days (1 year) in the absence of treatment, said medicament would increase their average life expectancy by about 10% of 365 days or more, for a total of about 400 days total survival.

By "soluble TACI reagent" means a soluble form of a TACI protein or polypeptide fragment in which the transmembrane domain has been cleaved or mutated by standard biochemical or recombinant DNA techniques such that it is soluble. Therefore, the invention provides use of a TACI reagent in the manufacture of a medicament for the prevention or treatment of a solid tumor, wherein said medicament extends the mean survival time and/or reduces solid tumor size, wherein said TACI reagent can augment or diminish ligand binding to TACI and wherein said TACI reagent is a fusion polypeptide comprising at least two segments, wherein a first segment comprises a TACI polypeptide or fragment thereof selected from the group consisting of:
(a) amino acid residues 1-166 of SEQ ID NO:1;
(b) amino acid residues 1-160 of SEQ ID NO: 1;
(c) amino acid residues 1-114 of SEQ ID NO: 1;
(d) amino acid residues 32-114 of SEQ ID NO: 1;
(e) a polypeptide having at least 80% amino acid sequence identity with amino acid residues 1-166 of SEQ ID NO: 1;
(f) a polypeptide having at least 80% amino acid sequence identity with amino acid residues 1-160 of SEQ ID NO: 1;
(g) a polypeptide having at least 80% amino acid sequence identity with amino acid residues 1-114 of SEQ ID NO: 1; and
(h) a polypeptide having at least 80% amino acid sequence identity with amino acid residues 32-114 of SEQ ID NO:1, and
a second segment comprises an immonoglobulin polypeptide.

The claimed invention in certain embodiments includes the use of fragments of TACI which have the ability to bind to APRIL. Fragments of TACI can be produced in several ways, *e.g.*, recombinantly, by PCR, proteolytic digestion or by chemical synthesis. Internal or terminal fragments of a polypeptide can be generated by removing one or more nucleotides from one end or both ends of a nucleic acid that encodes the polypeptide. Expression of the mutagenized DNA produces polypeptide fragments.

Polypeptide fragments can also be chemically synthesized using techniques known in the art such as conventional Merrifield solid phase f-moc or t-boc chemistry. For example, peptides and DNA sequences of the present invention may be arbitrarily divided into fragments of desired length with no overlap of the fragment, or divided into overlapping fragments of a desired length. Methods such as these are described in more details below.

Soluble forms of the TACI can often signal effectively and, hence, can be administered as a drug which now mimics die natural membrane form. It is possible that the TACI recited herein are naturally secreted as soluble cytokines, however, if not, one can reengineer the gene to force secretion. To create a soluble secreted form of TACI, one would remove at the DNA level the N-terminus transmembrane regions, and some portion of the stalk region, and replace them with a type I leader or alternatively a type II leader sequence that will allow efficient proteolytic cleavage in the chosen expression system. A skilled artisan could vary the amount of the stalk region retained in the secretion expression construct to optimize both ligand binding properties and secretion efficiency. For example, the constructs containing all possible stalk lenghts, i.e., N-terminal truncations, could be prepared. In certain embodiments, proteins starting at amino acids 1 to 32 are produced. The optimal length stalk sequence would result from this type of analysis.

By "substantially pure" or "substantially purified" is meant a compound (*e.g.*, a nucleic acid molecule or a protein) that has been separated from components (*e.g.*, nucleic acid molecules, proteins, lipids, and/or carbohydrates) which naturally accompany it. Water, buffers, and other small molecules (*e.g.*, molecules having a molecular weight of less than about 1000 daltons) may accompany a substantially pure compound of the invention. Preferably, a substantially purified compound is at least 70%, by weight, free from components which naturally accompany it. More preferably, a substantially purified compound is at least 75%, by weight, free from components which naturally accompany it; still more preferably, at least 80%, by weight, free; even more preferably, at least 85%, by weight, free; and even more preferably, at least 90%, by weight, free from components which naturally accompany it Most preferably a substantially purified compound is at least 95%, by weight, free from components which naturally accompany it. In certain embodiments of the

The "TACI extracellular domain" refers to a form of a TACI protein or polypeptide which is free of transmembrane and cytoplasmic domains of TACI. Ordinarily, TACI extracellular domain is amino acids 1 to about 166 of SEQ ID NO: 1. It is understood by the skilled artisan that the transmembrane domain identified for the TACI protein or polypeptide fragment of the present invention is identified pursuant to criteria routinely employed in the art for identifying that type of hydrophobic domain. The exact boundaries of a transmembrane domain vary but most likely by no more than about five amino acids at either end of the domain specifically mentioned herein.

"Sequence identity" with respect TACI amino acid sequences identified herein is defined as the percentage of amino acid residues in a candidate sequence that are identical with the amino acid residues in the TACI amino acid sequence, after aligning the sequences and introducing gaps, if necessary, to achieve the maximum percent sequence identity, and not considering any conservative substitutions as part of the sequence identity. Alignment for purposes of determining percent amino acid sequence identity is achieved in various ways that are within the skill in the art, for instance, using publicly available computer software such as BLAST, ALIGN, or Megalign (DNASTAR) software. Those skilled in the art determine appropriate parameters for measuring local alignment, including any algorithms needed to achieve maximum alignment over the full length of the sequences being compared, to detect relationships among sequences which share only isolated regions of similarity. (Altschul *et al.* (1990) *J. Mol. Biol.* 215:403-410).

By "fusion protein" means a protein that comprises at least two segments of a protein or polypeptide fragment joined together by any means, including, without limitation, a covalent bond (*e.g.,* peptide bond), a non-covalent bond (*e.g.,* ionic bond or hydrogen bond) or by a chemical crosslinker. Also, any variety of fusion proteins carrying only the extracellular domain of the TACI protein can be generated. Non-limiting examples include a fusion protein comprising the extracellular domain of the TACI protein and an immunoglobulin polypeptide, including for example, the immunoglobulin polypeptide IgG.

In addition, standard recombinant DNA techniques can be used to alter the binding affinities of recombinant antibodies with their antigens by altering amino acid residues in the vicinity of the antigen binding sites. The antigen binding affinity of a humanized antibody can be increased by mutagenesis based on molecular modeling. (Queen *et al.,* (1989) *Proc. Natl. Acad*. *Sci. USA* 86:10029-10033) incorporated herein by reference.

The medicament comprising a TACI polypeptide may include a pharmaceutically acceptable excipient. Suitable carriers for a the medicament for instance, and their formulations, are described in REMINGTON' PHARMACEUTICAL SCIENCES, 16^{th} ed., Oslo *et al.* Eds., Mack Publishing Co., 1980. Typically, an appropriate amount of a pharmaceutically acceptable salt is used in the formulation to render the formulation isotonic. Examples of the carrier include buffers such as saline, Ringer's solution and dextrose solution. The pH of the solution is preferably from about 5 to about 8, and more preferably from about 7.4 to about 7.8. Further carriers include sustained release preparations such as semipermeable matrices of solid hydrophobic polymers, which matrices are in the form of shaped articles, *e.g.*, liposomes, films, or microparticles. It will be apparent to those af skill in the art that certain carriers may be more preferable depending upon for instance the route of administration and concentration of the medicament being administered.

Administration may be accomplished by injection (*e.g.,* intravenous, intraperitoneal, subcutaneous, intramuscular) or by other methods such as infusion that ensure delivery to the bloodstream an effective form.

The medicaments comprising TACI reagents are administered in an effective amount which may easily be extrapolated by the animal data provided herein by methods known to those of ordinary skill in the art (*e.g.*, based on body weight, body surface area). Furthermore, it is in the purview of the skilled physician to increase or decrease amounts of the medicament comprising a TACI reagent to achieve the desired effects without causing any undesirable side effects.

The following Examples are provided to illustrate the present invention, and should not be construed as limiting thereof.

### EXAMPLES

The following methods and materials were used in the Examples disclosed hereinafter.

### I. Methods and Materials

### A. Cloning and expression of myc-tagged A88 murine APRIL in Pichia pastoris

The expression vector pCCM276 (Figure 4), was constructed by polymerase chain reaction using pCCM213.10 (Myc-tagged-H98 muAPRIL) as template and synthetic oligonucleotides CDL620 and LTB619 as forward and reverse primers, respectively. Forward primer CDL620 adds back 10 amino acids of murine APRIL sequence to represent the native cleaved form, along with a FAS ligand derived KEL motif and Sacl site. Resultant amplified fragments were digested with *Sac*l and *Not*l*,* gel purified and ligated into those same restriction sites of pCCM2I3.10. This expression construct contains yeast signal sequence alpha mating factor directly fused to myc epitope tag, KEL motif and murine APRIL starting at Alanine 88. pCCM2786 was linearized with *Stu*I, electroporated into GS115 strain (his4-) and plated onto minimal media containing dextrose. HIS4 transformants were analyzed for protein expression by inoculating a single representative colony in rich media (BMGY) and allowing it to grow to density for 48 hours at 30°C. Cultures were spun, and cell pellets were resuspended (1:5) in a rich induction media containing 2% methanol (BMMY). After two days of induction at 30°C, supernates were run out on SDS-PAGE and assessed for the presence of muAPRIL. Coomassie blue staining and Western Blot (with the anti-myc mAB 9E10) analysis confirmed the presence of the glycosylated A88 myc-tagged murine APRIL.

### B. Myc-Murine APRIL A88 Purification

Myc-tagged murine April (myc-muAPRIL) was expressed in *P. pastoris.* The protein has an isoelectric point of about 7.45. 175 ml of *Pichia* supernate was dialyzed into PBS overnight with a 30kD cutoff dialysis membrane. The dialysate was then passed through a Q sepharose column. The myc-muAPRIL was collected in the column flow through, while contaminants were bound to the column. The eluted myc-muAPRIL was concentrated 20 fold and then chromatographed on a superdex 75 gel filtration column. After gel filtration, 8 mg of myc-muAPRIL were recovered having an OD of 1.0AU-1 mg of myc-muAPRIL. Coomassie stained SDS PAGE showed a homogenous preparation of myc-muAPRIL with two bands migrating at molecular weights of approximately 22 kD and 18 kD. Western blot analysis using mouse monoclonal 9E10 antibody (anti-myc) showed that both bands observed were immunoreactive. The expected N-terminus of the purified myc-muAPRIL was verified by Edman degradation of the blotted protein. The N-terminal sequencing and immunoreactivity to 9E 10 prove the myc tag was present on the myc-muAPRIL N-termini.

### C. FLAG-Murine APRIL Purification

Plasmid PS784 (also called LT032) (Figure 7) was used to transiently transfect 293 T cells using lipofectamine reagent (Gibco-BRL) and serum free media. The plasmid, constructed in the mammalian expression vector PCR3 (Invitrogen) encodes the soluble receptor-binding domain of murine APRIL, with an N-terminal FLAG-tag, into the cell culture media. FLAG-APRIL protein was purified from serum free media using an anti-FLAG mAb M2 column and excess purified FLAG peptide, following the manufacturers' instructions (Kodak). Alternatively murine APRIL and other FLAG-tagged ligands were analyzed directly from conditioned media.

### D. hTACI(1-160)-Ig Expression

A plasmid encoding soluble hTACI(1-160)-Ig (PS882; Figure 5) was used to transiently to transfect 293 cells. Conditioned media from 293 cells overexpressing hTACI(1-160)-Ig was used in the immunoprecipitation analysis to detect binding to APRIL protein.

### E. Full Length TACI Expression

A plasmid encoding flag-tagged full length TACI (pJST552; Figure 2) was transiently transfected into 293 cells. The full length form of the molecule is retained on the cell surface. These transfected cells were used in FACS analyses to detect binding to APRIL protein.

### F. FLAG-human APRIL production

Plasmid LT033 (Figure 9) was used to transiently transfect 293 T cells using Lipofectamine reagent (Gibco-BRL) and serum-free media. The plasmid, constructed in mammalian expression vector PCR3 (Invitrogen) encodes the soluble receptor-binding domain of human APRIL, with an N-terminal FLAG-tag, and the expressed protein is secreted into the cell culture media. FLAG human APRIL was analyzed directly from cell culture supemate.

### EXAMPLE 1

### Human TACI Expressed on Cells Interacts With Myc-Tagged Murine APRIL

293EBNA cells were co-transfected with a plasmids expressing full length N-terminally FLAG tagged human TACI (pJST552)(see Figure 2), and a GFP marker, (AN050). Transfections were performed using Lipofectamine 2000 (LifeTechnologies) according to manufacturer's conditions. Transfection media was DMEM supplemented with 10% FBS, 4 mM glutamine, and 50 µM Z-VAD (BACHEM Bioscience Inc.) Cells were harvested with PBS supplemented with 5 mM EDTA at 24 hours post-transfection. Cells were washed in FACS buffer (PBS- 10% FBS-0.02% NaN₃) and resuspended at a density of 5x10⁶ cells/mL. Expression of TACI was verified by staining 100 microliters of transfected cells with anti-FLAG mAb at 5µg/ml on ice for 30 minutes followed by donkey anti-mouse IgG-PE at 1:100 (Jackson ImmunoResearch). Cells were assayed for their ability to bind APRIL over a concentration range of 3 µg/ml to 300 ng/ml by incubating on ice in FACS buffer for 30 minutes. Binding was revealed using Rb1532, a rabbit polyclonal Ab raised to mAPRIL (1:100), followed by donkey anti-rabbit IgG-PE (1:100, Jackson ImmunoResearch). 7-AAD was included in the terminal stain and used to gate out dead cells. The samples were analyzed by FACS and plotted (Figure 8). Cell gate analyzed is shown as R1. Specific staining is seen at the three different concentrations of myc-tagged murine APRIL protein shown, as compared to no protein control (Figure 8).

### EXAMPLE 2

### Soluble hTACI(1-160)-Ig Interacts with FLAG-Tagged Murine APRIL

250 *µ*l of Optimem containing hTACI(1-160)-Ig was mixed with various FLAG ligands of the TNF family. Some of the ligands were in Optimem while others were anti-FLAG (mAb M2)-purified. The common characteristic was that the amount used was empirically the same (500 ng for purified ligands, *i.e*. hBAFF, muBAFF, TRAIL, FasL, EDA). Ligands were produced in bacteria (hBAFF, TRAIL) or transiently expressed in 293 EBNA cells (all others). Control Receptors-Fc were mostly used as Optimem supernates, except TNFR1, OX40, LTBR, which were purified over protein-A columns. 1 *µ*g of purified receptors was used. Receptor-Fc proteins (about 1 *µ*g) were mixed with FLAG-ligands (about 500 ng). Volume was adjusted to 1.2 ml with PBS and 5 *µ*l of protein A-Sepharose was added. Incubation was performed for 1 h at 4°C on a wheel. Beads were harvested by centrifugation and loaded onto a minicolumn (yellow tips with 1 mm diameter frit). Washes were performed by applying vacuum at the bottom of the column to aspirate medium and 2 times 400 *µ*l PBS washes. The dried beads were eluted with 20 *µ*l of Citrate -NaOH pH 2.7 and the eluate was neutralized with 6 *µ*l of 1M Tris-HCl pH 9.0. 10 *µ*l 3x sample buffer plus DTT was added, the sample was boiled and 22 *µ*l loaded for Western blot analysis. The membrane was sequentially stained with Ponceau-S, blocked in 4% milk, 0.5% Tween-20, then incubated with 1 *µ*g/ml M2 in blocking buffer, washed, and revealed with goat anti-mouse-Ig-peroxidase (1/5000 in block buffer). ECL was used to illuminate the signal. Peroxidase activity was removed with azide, the membrane washed, and then probed again with donkey anti-human-peroxidase.

The western results of the co-immunoprecipitation show that only flag-muAPRIL is able to be co-immunoprecipitated by TACI(1-160)-Ig. The binding of TACI(1-160)-Ig to muAPRIL appears to be specific as none of the other 14 flag tagged soluble TNF family ligands were able to interact with TACI(1-160)-Ig.

### EXAMPLE 3

### Use of Derived TACI Sequence, and Antagonists and Agonists of TACI Binding and Activity, as Modifiers of Oncological and Neoplastic Disorders

Examples of the preparation and inoculation of transformed cells to assess tumor cell growth is described in Celis *et al.,* CELL BIOLOGY, A LABORATORY HANDBOOK, Volume One, Academic Press, San Diego, CA. 1997. Tumor cells were derived from a variety of sources, including the extensive tumor cell banks maintained by ATCC (Bethesda, MD), immortalized cell lines, immortalized primary cell lines, stably transfected cell lines, tumor tissue derived from mammalian sources, including humans. Tissue source is a major determinant of choice of immunodeficient or normal animals (Celis *et al., ibid.).* In addition there are a wide variety of techniques for the induction of tumor growth in various animal models using carcinogenic or other insults.

Models which utilize tumor growth in immunodeficient mice as a means of readily determining the activity of novel ligands on tumor biology (Hahne *et al.* (1998) *J. Exp. Med.* 188:1185-1190) were developed. Both ligands and their antagonists were assayed in such systems, *e.g.,* Kashii *et al.* (1999) *J. Immunol.* 163:5358-5366; Zhai *et al.* (1999) *FASEB J.* 13:181-189). An agonist mAb to a TNF receptor family member (LTBR) profoundly affected tumor cell growth and survival was demonstrated (Browning *et al.* (1996) *J. Exp. Med.* 183:867-878).

Tumor cell lines were implanted in immunodeficient mice subcutaneously, and the growth rate of tumors in mice treated with TACI-Ig was similar to the growth rate of tumors in mice treated with approximately Smg/kg/week CBE11, that is, much slower tumor growth as compared to the growth rate of mice given control treatments.

Another variation of these models was to use as a tumor source cells known to induce metastasis in immunodeficient or syngeneic animals. For example, the ATCC (Bethesda, MD) provided numerous human tumor lines with known metastatic potential to a variety of tissues, and these lines are utilized to examine the effect of TACI activity or antagonism on metastasis. These models have been and are now currently used (*e.g.*, by the NCI) to assess the potential for treatment of human patients.

### EXAMPLE 4

### Generation of Soluble TACI Receptors

To form a receptor inhibitor for use in man, the human receptor cDNA sequence of the extracellular domain of TACI was used (SEQ ID NO:2). With a human cDNA sequence, oligonucleotide primers were designed to PCR amplify the extracellular domain of the receptor in the absence of the transmembrane and intracellular domains. Typically, one of ordinary skill included most of the amino acids between the last disulfide linked "TNF domain" and the transmembrane domain. Alternatively, the amount of "stalk" region was varied to optimize the potency of the resultant soluble receptor. This amplified piece was engineered to include suitable restriction sites to allow cloning into various C-terminal Ig fusion chimera vectors. Alternatively, a stop signal at the 3' end was inserted to make a soluble form of the receptor without resorting to the use of an Ig fusion chimera approach. The resultant vectors were expressed in most systems used in biotechnology including yeast, insect cells, bacteria and mammalian cells and examples exist for all types of expression. Various human Fc domains were attached to optimize or eliminate FcR and complement interactions as desired. Alternatively, mutated forms of these Fc domains were used to selectively remove FcR or complement interactions or the attachment of N-linked sugars to the Fc domain which has certain advantages.

### EXAMPLE 5

### Screening for Inhibitors of the Receptor-Ligand Interaction

Using the receptor-Ig fusion protein, one screens either combinatorial libraries as screened for molecules that bind the receptor directly. These molecules are then tested in an ELISA formatted assay using the receptor-Ig fusion protein and a soluble form of the ligand for the ability to inhibit the receptor-ligand interaction. This ELISA is used directly to screen various natural product libraries, *etc.* for inhibitory compounds. The receptor is transfected into a cell line such as the HT29 line to form a biological assay (in this case cytotoxicity) that then form the screening assay to further demonstrate blocking.

### EXAMPLE 6

### In vivo Tumor Growth Inhibition

The number of tumor cells injected subcutaneously (s.c.) can be determined in titration studies prior to initiating work with antagonists. For example, the SW480 - colon adenocarcinoma solid tumor line and NIH 3T3 fibrosarcoma line, which grow aggressively, 8 x 10⁵ cells and 5 x 10⁶ cells, respectively, can be implanted in nude mice. Dosing with control or TACI-Ig proteins can begin just prior to implantation, with subsequent doses every 7 days thereafter. The dose can be for example 100 *µ*g/mouse. Tumor diameter is then measured using a micrometer, and the volume is calculated using the formula vol = 4/3πr³.

### EXAMPLE 7

### In vivo Tumor Growth Inhibition

The number of tumor cells injected s.c. into immunodeficient (nu/nu) mice was determined in previous dose response studies. For the studies using HT29, 1 x 10⁶ cells were implanted per mouse. HT29 is derived from a human colon adenocarcinoma, and has similar growth characteristics to some other human colon adenocarcinoma lines (eg., SW480) such as rapid tumor formation, and rapid tumor growth. Mice implanted with HT29 cells were treated on the day of implantation, and every week thereafter, with 100ugs hTACI(1-114)-Ig given intraperitoneally (i.p.). Negative controls included the irrelevant proteins polyclonal hIgG and mAb MOPC21. Positive controls included BCMA-Ig, another inhibitor of APRIL binding, and CBE11, a mAb to the mLTb-R which is known to slow adenocarcinoma tumor growth (Browning *et al.* (1996) *J. Exp. Med.* 183:867-878). Tumor diameter was measured using a micrometer, and the volume was calculated using the formula vol = 4/3πR³.

For studies with the lung carcinoma A549 we implanted 1 x 10⁶ cells/mouse. Mice were treated with 100 *µ*g TACI(1-114)-Ig, 100 *µ*g BCMA-Ig, 100 *µ*g hIgG, or 100 µl PBS starting on the day of implantation and treated weekly thereafter. Tumor diameter was measured using a micrometer, and the volume was calculated using the formula vol = 4/3πR³.

The results showing tumor,growth inhibition of the HT29 colon adenocarcinoma are shown in Figure 11. The results showing tumor growth inhibition of the A549 lung carcinoma are shown in Figure 12. In both experiments, significant slowing of tumor growth was achieved. Since tumor size and survival are directly linked in these models, TACI-Ig treatment also impacted animal survival. For example, 95 days after tumor implantation, 50% of the hIgG treated mice were scored as terminal, compared to only 12.5% of the TACI-Ig treated mice. This represents a 4 fold increase in survival at this end stage time point.

### EXAMPLE 8

### hTACI(1-114)-Ig and hTACI(32-114)-Ig bind to surface localized APRIL

This example shows titratable binding of TACI-Ig fusion proteins a stable cell line expressing muAPRIL on their surface. Either 25 µl or 5 µl of conditioned media from 293EBNA cells transiently transfected with plasmids expressing hTACI(1-114)-Ig, hTACI(32-114) or hFN14-Fc were diluted in FACS buffer to a final volume of 50 µl and incubated for 1 hour on ice with 2.5 x 10⁵ 293 cells stably expressing the receptor binding domain of muAPRIL on their surface. After washing with FACS buffer, cells were then incubated with anti-human IgG-PE (1:100 dilution, Jackson ImmunoResearch) for 30 minutes on ice. Cells were again washed, fixed in 1% paraformaldehyde and analyzed by FACS. Figure 13 shows an FL2 shift indicating titratable staining of surface muAPRIL by HTACI(1-114)-Ig and hTACI(32-114)-Ig. No staining was observed with the second step only control. No staining was observed with either dilution of a control-Ig, FN14-Ig fusion protein, both of which co-migrate with the second step only histogram.

### EXAMPLE 9

### Binding of murine and human APRIL to hTACI(32-114)-Ig in ELISA format

ELISA plates (Coming) were coated with 5 ug/ml hTACI(32-114)-Ig or mLTbR-Ig control in bicarbonate buffer pH = 9.6 overnight, then washed in PBS/0.05% Tween-20 solution before blocking for 2 h at 37°C with PBS/2% bovine serum albumin (BSA). APRIL protein preparations were added in the range of 0.0048 to 3 µg/ml, diluted in PBS/2% BSA. Detection of specific APRIL binding was with rabbit anti-murine APRIL antisera (R1532) and donkey anti-rabbit HRP (Jackson Immunoresearch) or with HRP-coupled anti FLAG mAb M2 (Kodak). Enzymatic development was done with TMA and H₂O₂ and stopped with H₂SO₄, following standard protocols. The developed yellow stain was read at 450 nm on a plate reader. The results are shown in Figure 14.

### EXAMPLE 10

### Preferential Expression of APRIL in Tumor Samples

APRIL mRNA is preferentially expressed in tumor samples. Analysis of the cDNA libraries collected by Incyte Inc., and shown in Table I, demonstrates that APRIL is widely expressed in numerous solid tumor and neoplastic samples, but much less often expressed in normal tissue samples. Expression in diseased tissue is occasionally prominent.

**Table I**

| **EXPRESSION OF APRIL IN TISSUES (INCYTE)** | | |
|---|---|---|
| **PROSTATE/BLADDER** | **No. +** | **DISTRIBUTION** |
| Normal | 5 | |
| Tumor | 11 | adenocarcinoma |

| **COLON** | | |
|---|---|---|
| Normal | 0 | |
| Tumor | 6 | adenocarcinoma |
| Diseased | 4 | 2 Crohn's Disease |
| | | 1 ulcerative colitis |
| | | 1 polyp |

| **BREAST** | | |
|---|---|---|
| Normal | 1 | normal epithelium |
| Tumor | 11 | 5 adenocarcinoma |
| | | 3 ductal carcinoma |
| | | 1 lobule carcinoma |
| | | 2 unspecified |

| **PANCREAS** | | |
|---|---|---|
| Normal | 0 | |
| Tumor | 5 | 1 anaplastic carcinoma |
| | | 4 adenocarcinoma |

| **LUNG** | | |
|---|---|---|
| Normal | 2 | 4 adenocarcinoma |
| Tumor | 12 | 4 adenocarcinoma |
| | | 3 squamous cell carcinoma |
| | | 3 carcinomoid |
| | | spindle cell |
| | | endobonchial |
| | | neuroendocrine |
| | | 2 metastases |
| Diseased | 7 | |

| **UTERUS/OVARY** | | |
|---|---|---|
| Normal | 0 | |
| Tumor | 13 | 3 cystadenocarcinoma |
| | | 2 endometrial tumors |
| | | 4 leiomyomata |
| | | 2 adenocarcinoma |
| | | 1 papillary carcinoma |
| | | 1 metastases |
| Diseased | 1 | 1 ovarian cyst |

| **Other Normal Tissues** | | |
|---|---|---|
| MO/DC | 10 | |
| **Endothelial** | 5 | 2 arterial endothelium |
| | | 1 dermal endothelium |
| | | 1 arterial smooth muscle |
| | | 1 arterial plaque |
| **Other Hyperplastic Sites** | 6 | 2 lymphomas |
| | | 2 lymphoid hyperplasia |
| | | 2 rheumatoid arthritis synovium |
| | | 1 hyperthyroid |
| | | 1 nasal polyp |

## Claims

1. Use of a TACI reagent in the manufacture of a medicament for the prevention or treatment of a solid tumor, wherein said medicament extends the mean survival time and/or reduces solid tumor size, wherein said TACI reagent can augment or diminish ligand binding to TACI and wherein said TACI reagent is a fusion polypeptide comprising at least two segments, wherein a first segment comprises a TACI polypeptide or fragment thereof selected from the group consisting of:
(a) amino acid residues 1-166 of SEQ ID NO:1;
(b) amino acid residues 1-160 of SEQ ID NO:1;
(c) amino acid residues 1-114 of SEQ ID NO: 1;
(d) amino acid residues 32-114 of SEQ ID NO:1;
(e) a polypeptide having at least 80% amino acid sequence identity with amino acid residues 1-166 of SEQ ID NO: 1;
(f) a polypeptide having at least 80% amino acid sequence identity with amino acid residues 1-160 of SEQ ID NO: 1;
(g) a polypeptide having at least 80% amino acid sequence identity with amino acid residues 1-114 of SEQ ID NO:1; and
(h) a polypeptide having at least 80% amino acid sequence identity with amino acid residues 32-17.4 of SEQ ID NO:1, and
a second segment comprises an immunoglobulin polypeptide.

2. The use of claim 1 wherein the mean survival time is extended by up to about 15%.

3. The use of claim 1 wherein the mean survival time is extended by up to about 20%.

4. The use of claim 1 wherein the mean survival time is extended by up to about 25%.

5. The use of any one of claims 1 to 4 wherein said solid tumor is a cancer selected from renal cell cancer, Kaposi's sarcoma, breast cancer, sarcoma, ovarian carcinoma, rectal cancer, throat cancer, melanoma, colon cancer, bladder cancer, mastocytoma, lung cancer, mammary adenocarcinoma, pharyngeal squamous cell carcinoma, gastrointestinal cancer or stomach cancer.

6. The use of any one of claims 1 to 4 wherein said solid tumor is suffered by a human, a cow, a horse, a dog, a mouse, a rat or a cat.

7. The use of claim 1 wherein said solid tumor is reduced in size by up to about 10%.

8. The use of claim 1 wherein said solid tumor is reduced in size by up to about 15%.

9. The use of claim 1 wherein said solid tumor is reduced in size by up to about 20%.

10. The use of claim 1 wherein said solid tumor is reduced in size by up to 25% or more.

11. The use of any one of claims 1 to 10 wherein said immunoglobulin polypeptide is an Fc domain.

12. The use of claim 11 wherein said Fc domain is an IgG Fc domain.

## Patentansprüche

1. Verwendung eines TACI-Reagenzes zur Herstellung eines Medikaments zur Vorbeugung oder Behandlung eines festen Tumors, worin das Medikament die mittlere Überlebenszeit verlängert und/oder die Größe eines festen Tumors vermindert, worin das TACI-Reagenz Ligandenbindung an TACI verstärken oder vermindern kann und worin das TACI-Reagenz ein Fusionspolypeptid ist, das wenigstens zwei Segmente umfasst, wobei ein erstes Segment ein TACI-Polypeptid oder Fragment davon umfasst, gewählt aus der Gruppe, die besteht aus:
(a) Aminosäureresten 1-166 von SEQ-ID-Nr.: 1;
(b) Aminosäureresten 1-160 von SEQ-ID-Nr.: 1;
(c) Aminosäureresten 1-114 von SEQ ID Nr.: 1;
(d) Aminosäureresten 32-114 von SEQ-ID-Nr 1;
(e) einem Polypeptid mit wenigstens 80% Aminosäuresequenzidentität mit Aminosäureresten 1-166 von SEQ-ID-Nr.: 1;
(f) einem Polypeptid mit wenigstens 80% Aminosäuresequenzidentität mit Aminosäureresten 1-160 von SEQ-ID-Nr.: 1;
(g) einem Polypeptid mit wenigstens 80% Aminosäuresequenzidentität mit Aminosäureresten 1-114 von SEQ ID Nr.: 1; und
(h) einem Polypeptid mit wenigstens 80% Aminosäuresequenzidentität mit Aminosäureresten 32-114 von SEQ-ID-Nr.: 1, und
ein zweites Segment, das ein Immunoglobulinpolypeptid umfasst.

2. Verwendung nach Anspruch 1, wobei die mittlere Überlebenszeit um bis zu etwa 15% verlängert wird.

3. Verwendung nach Anspruch 1, wobei die mittlere Überlebenszeit um bis zu etwa 20% verlängert wird.

4. Verwendung nach Anspruch 1, wobei die mittlere Überlebenszeit um bis zu etwa 25% verlängert wird.

5. Verwendung nach einem der Ansprüche 1 bis 4, wobei der feste Tumor gewählt ist aus Nierenzellkrebs, Kaposi-Sarkom, Brustkrebs, Sarkom, Eierstockkrebs, Rektalkrebs, Kehlkopfkrebs, Melanom, Darmkrebs, Blasenkrebs, Mastozytom, Lungenkrebs, Brustdrüsen-Adenokarzinom, squamöses Rachenzellkarzinom, Gastrointestinalkrebs und Magenkrebs.

6. Verwendung nach einem der Ansprüche 1 bis 4, wobei der feste Tumor von einem Mensch, einer Kuh, einem Pferd, einem Hund, einer Maus, einer Ratte oder einer Katze erlitten wird.

7. Verwendung nach Anspruch 1, wobei der feste Tumor in der Größe um bis zu etwa 10% verringert ist.

8. Verwendung nach Anspruch 1, wobei der feste Tumor in der Größe um bis zu etwa 15% verringert ist.

9. Verwendung nach Anspruch 1, wobei der feste Tumor in der Größe um bis zu etwa 20% verringert ist.

10. Verwendung nach Anspruch 1, wobei der feste Tumor in der Größe um bis zu etwa 25% oder mehr verringert ist.

11. Verwendung nach einem der Ansprüche 1 bis 10, wobei das Immunoglobulinpolypeptid eine Fc-Domäne ist.

12. Verwendung nach Anspruch 11, wobei die Fc-Domäne eine IgG Fc-Domäne ist.

## Revendications

1. Utilisation d'un réactif TACI dans la fabrication d'un médicament pour la prévention ou le traitement d'une tumeur solide, le médicament prolongeant la durée moyenne de survie et/ou réduisant la dimension de la tumeur solide, le réactif TACI pouvant augmenter ou diminuer la fixation ligandaire au TACI et le réactif TACI étant un polypeptide de fusion comprenant au moins deux segments, dans laquelle un premier segment comprend un polypeptide TACI ou l'un de ses fragments choisi dans le groupe consistant en :
(a) des résidus d'acide aminé 1 à 166 du n° 1 d'identification des séquences,
(b) des résidus d'acide aminé 1 à 160 du n° 1 d'identification des séquences,
(c) des résidus d'acide aminé 1 à 114 du n° 1 d'identification des séquences,
(d) des résidus d'acide aminé 32 à 114 du n° 1 d'identification des séquences,
(e) un polypeptide ayant au moins une identité à 80 % de la séquence d'acide aminé avec des résidus d'acide aminé 1 à 166 du n° 1 de l'identité des séquences,
(f) un polypeptide ayant au moins une identité à 80 % de la séquence d'acide aminé avec des résidus d'acide aminé 1 à 160 du n° 1 de l'identité des séquences,
(g) un polypeptide ayant au moins une identité à 80 % de la séquence d'acide aminé avec des résidus d'acide aminé 1 à 114 du n° 1 de l'identité des séquences et
(h) un polypeptide ayant au moins une identité à 80 % de la séquence d'acide aminé avec des résidus d'acide aminé 32 à 114 du n° 1 de l'identité des séquences et
un deuxième segment comprend un polypeptide d'immunoglobuline.

2. L'utilisation suivant la revendication 1, dans laquelle la durée moyenne de survie est prolongée jusqu'à 15 % environ.

3. L'utilisation suivant la revendication 1, dans laquelle la durée moyenne de survie est prolongée jusqu'à 20 % environ.

4. L'utilisation suivant la revendication 1, dans laquelle la durée moyenne de survie est prolongée jusqu'à 25 % environ.

5. L'utilisation suivant l'une quelconque des revendications 1 à 4, dans laquelle la tumeur solide est un cancer choisi parmi le cancer des cellules rénales, le sarcome de Kaposi, le cancer du sein, le sarcome, le carcinome de l'ovaire, le cancer du rectum, le cancer de la gorge, le mélanome, le cancer du colon, le cancer de la vessie, le mastocytome, le cancer du poumon, l'adénocarcinome mammaire, l'épithéliome malpighien spinocellulaire du pharynx, le cancer du tube digestif ou le cancer de l'estomac.

6. L'utilisation suivant l'une quelconque des revendications 1 à 4, dans laquelle un homme, une vache, un cheval, un chien, une souris, un rat ou un chat souffre de la tumeur solide.

7. L'utilisation suivant la revendication 1, dans laquelle la tumeur solide est réduite en dimension jusqu'à environ 10 %.

8. L'utilisation suivant la revendication 1, dans laquelle la tumeur solide est réduite en dimension jusqu'à environ 15 %.

9. L'utilisation suivant la revendication 1, dans laquelle la tumeur solide est réduite en dimension jusqu'à environ 20 %.

10. L'utilisation suivant la revendication 1, dans laquelle la tumeur solide est réduite en dimension jusqu'à 25 % ou plus de 25 %.

11. L'utilisation suivant l'une quelconque des revendications 1 à 10, dans laquelle le polypeptide d'immunoglobuline est un domaine Fc.

12. L'utilisation suivant la revendication 11, dans laquelle le domaine Fc est un domaine d'IgFc.


### SEQUENCE LISTING

<110> Biogen, Inc.
<120> Taci As As Anti-Tumor Agent
<130> BIOG0078
<140> PCT/US01/40626
   <141> 2001-04-27
<150> USSN 60/199,946
   <151> 2000-04-27
<160> 17
<170> PatentIn version 3.1
<210> 1
   <211> 293
   <212> PRT
   <213> Homo sapiens
<400> 1
<210> 2
   <211> 882
   <212> DNA
   <213> Homo sapiens
<400> 2
   atgagtggcc tgggccggag caggcgaggt ggccggagcc gtgtggacca ggaggagcgc      60
   tttccacagg gcctgtggac gggggtggct atgagatcct gccccgaaga gcagtactgg     120
   gatcctctgc tgggtacctg catgtcctgc aaaaccattt gcaaccatca gagccagcgc     180
   acctgtgcag ccttctgcag gtcactcagc tgccgcaagg agcaaggcaa gttctatgac     240
   catctcctga gggactgcat cagctgtgcc tccatctgtg gacagcaccc taagcaatgt     300
   gcatacttct gtgagaacaa gctcaggagc ccagtgaacc ttccaccaga gctcaggaga     360
   cagcggagtg gagaagttga aaacaattca gacaactcgg gaaggtacca aggattggag     420
   cacagaggct cagaagcaag tccagctctc ccggggctga agctgagtgc agatcaggtg     480
   gccctggtct acagcacgct ggggctctgc ctgtgtgccg tcctctgctg cttcctggtg     540
   gcggtggcct gcttcctcaa gaagaggggg gatccctgct cctgccagcc ccgctcaagg     600
   ccccgtcaaa gtccggccaa gtcttcccag gatcacgcga tggaagccgg cagccctgtg     660
   agcacatccc ccgagccagt ggagacctgc agcttctgct tccctgagtg cagggcgccc     720
   acgcaggaga gcgcagtcac gcctgggacc cccgacccca cttgtgctgg aaggtggggg     780
   tgccacacca ggaccacagt cctgcagcct tgcccacaca tcccagacag tggccttggc    840
   attgtgtgtg tgcctgccca ggaggggggc  ccaggtgcat aa                       882
<210> 3
   <211> 249
   <212> PRT
   <213> Mus musculus
<400> 3
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Asp Glu Thr Ala Gln
               20                  25                  30
   Ile Pro Ala Glu Ala Val Ile Gly Tyr Ser Asp Leu Glu Gly Asp Phe
           35                  40                  45
   Asp Val Ala Val Leu Pro Phe Ser Asn Ser Thr Asn Asn Gly Leu Leu
       50                  55                  60
   Phe Ile Asn Thr Thr Ile Ala Ser Ile Ala Ala Lys Glu Glu Gly Val
   65                  70                  75                  80
   Ser Leu Glu Lys Arg Glu Gln Lys Leu Ile Ser Glu Glu Asp Leu Asn
                   85                  90                  95
   Gly Gly Gly Gly Ser Lys Glu Leu Ala Val Leu Thr Gln Lys His Lys
               100                 105                 110
   Lys Lys His Ser Val Leu His Leu Val Pro Val Asn Ile Thr Ser Lys
           115                 120                 125
   Asp Ser Asp Val Thr Glu Val Met Trp Gln Pro Val Leu Arg Arg Gly
       130                 135                 140
   Arg Gly Leu Glu Ala Gln Gly Asp Ile Val Arg Val Trp Asp Thr Gly
   145                 150                 155                 160
   Ile Tyr Leu Leu Tyr Ser Gln Val Leu Phe His Asp Val Thr Phe Thr
                   165                 170                 175
   Met Gly Gln Val Val Ser Arg Glu Gly Gln Gly Arg Arg Glu Thr Leu
               180                 185                 190
   Phe Arg Cys Ile Arg Ser Met Pro Ser Asp Pro Asp Arg Ala Tyr Asn
           195                 200                 205
   Ser Cys Tyr Ser Ala Gly Val Phe His Leu His Gln Gly Asp Ile Ile
           210                 215                 220
   Thr Val Lys Ile Pro Arg Ala Asn Ala Lys Leu Ser Leu Ser Pro His
   225                 230                 235                 240
   Gly Thr Phe Leu Gly Phe Val Lys Leu
                   245
<210> 4
   <211> 788
   <212> DNA
   <213> Mus musculus
<400> 4
   gatccaaacg atgagatttc cttcaatttt tactgcagtt ttattcgcag catcctccgc      60
   attagctgct ccagtcaaca ctacaacaga agatgaaacg gcacaaattc cggctgaagc     120
   tgtcatcggt tactcagatt tagaagggga tttcgatgtt gctgttttgc cattttccaa     180
   cagcacaaat aacgggttat tgtttataaa tactactatt gccagcattg ctgctaaaga     240
   agaaggggta tctctcgaga aaagagaaca aaaactcatt tctgaggaag atctgaatgg     300
   tggcggtggg tccaaagagc tcgcagtact cacccagaag cacaagaaga agcactcagt     360
   cctgcatctt gttccagtta acattacctc caaggactct gacgtgacag aggtgatgtg     420
   gcaaccagta cttaggcgtg ggagaggcct ggaggcccag ggagacattg tacgagtctg     480
   ggacactgga atttatctgc tctatagtca ggtcctgttt catgatgtga ctttcacaat     540
   gggtcaggtg gtatctcggg aaggacaagg gagaagagaa actctattcc gatgtatcag     600
   aagtatgcct tctgatcctg accgtgccta caatagctgc tacagtgcag gtgtctttca     660
   tttacatcaa ggggatatta tcactgtcaa aattccacgg gcaaacgcaa aacttagcct     720
   ttctccgcat ggaacattcc tggggtttgt gaaactatga gcggccgcga attaattcgc     780
   cttagaca                                                              788
<210> 5
   <211> 1101
   <212> DNA
   <213> Homo sapiens
<220>
   <221> CDS
   <222> (1)..(1101)
   <223>
<400> 5
   atg gag aca gac aca ctc ctg tta tgg gtg ctg ctg ctc tgg gtt cca      48
   Met Glu Thr Asp Thr Leu Leu Leu Trp Val Leu Leu Leu Trp Val Pro
   1               5                   10                  15
   ggt tcc act ggt gac gtc acg atg agt ggc ctg ggc cgg agc agg cga      96
   Gly Ser Thr Gly Asp Val Thr Met Ser Gly Leu Gly Arg Ser Arg Arg
               20                  25                  30
   ggt ggc cgg agc cgt gtg gac cag gag gag cgc ttt cca cag ggc ctg      144
   Gly Gly Arg Ser Arg Val Asp Gln Glu Glu Arg Phe Pro Gln Gly Leu
           35                  40                  45
   tgg aca ggg gtg gct atg aga tcc tgc ccc gaa gag cag tac tgg gat      192
   Trp Thr Gly Val Ala Met Arg Ser Cys Pro Glu Glu Gln Tyr Trp Asp
       50                  55                  60
   cct ctg ctg ggt acc tgc atg tcc tgc aaa acc att tgc aac cat cag      240
   Pro Leu Leu Gly Thr Cys Met Ser Cys Lys Thr Ile Cys Asn His Gln
   65                  70                  75                  80
   agc cag cgc acc tgt gca gcc ttc tgc agg tca ctc agc tgc cgc aag      288
   Ser Gln Arg Thr Cys Ala Ala Phe Cys Arg Ser Leu Ser Cys Arg Lys
                   85                  90                  95
   gag caa ggc aag ttc tat gac cat ctc ctg agg gac tgc atc agc tgt      336
   Glu Gln Gly Lys Phe Tyr Asp His Leu Leu Arg Asp Cys Ile Ser Cys
               100                 105                 110
   gcc tcc atc tgt gga cag cac cct aag caa tgt gca tac ttc tgt gag      384
   Ala Ser Ile Cys Gly Gln His Pro Lys Gln Cys Ala Tyr Phe Cys Glu
           115                 120                 125
   aac aag ctc agg agc cca gtg aac ctt cca gtc gac aaa act cac aca      432
   Asn Lys Leu Arg Ser Pro Val Asn Leu Pro Val Asp Lys Thr His Thr
       130                 135                 140
   tgc cca ccg tgc cca gca cct gaa ctc ctg ggg gga ccg tca gtc ttc      480
   Cys Pro Pro Cys Pro Ala Pro Glu Leu Leu Gly Gly Pro Ser Val Phe
   145                 150                 155                 160
   ctc ttc ccc cca aaa ccc aag gac acc ctc atg atc tcc cgg acc cct      528
   Leu Phe Pro Pro Lys Pro Lys Asp Thr Leu Met Ile Ser Arg Thr Pro
                   165                 170                 175
   gag gtc aca tgc gtg gtg gtg gac gtg agc cac gaa gac cct gag gtc      576
   Glu Val Thr Cys Val Val Val Asp Val Ser His Glu Asp Pro Glu Val
               180                 185                 190
   aag ttc aac tgg tac gtg gac ggc gtg gag gtg cat aat gcc aag aca      624
   Lys Phe Asn Trp Tyr Val Asp Gly Val Glu Val His Asn Ala Lys Thr
           195                 200                 205
   aag ccg cgg gag gag cag tac aac agc acg tac cgt gtg gtc agc gtc      672
   Lys Pro Arg Glu Glu Gln Tyr Asn Ser Thr Tyr Arg Val Val Ser Val
       210                 215                 220
   ctc acc gtc ctg cac cag gac tgg ctg aat ggc aag gag tac aag tgc      720
   Leu Thr Val Leu His Gln Asp Trp Leu Asn Gly Lys Glu Tyr Lys Cys
   225                 230                 235                 240
   aag gtc tcc aac aaa gcc ctc cca gcc ccc atc gag aaa acc atc tcc      768
   Lys Val Ser Asn Lys Ala Leu Pro Ala Pro Ile Glu Lys Thr Ile Ser
                   245                 250                 255
   aaa gcc aaa ggg cag ccc cga gaa cca cag gtg tac acc ctg ccc cca      816
   Lys Ala Lys Gly Gln Pro Arg Glu Pro Gln Val Tyr Thr Leu Pro Pro
               260                 265                 270
   tcc cgg gat gag ctg acc aag aac cag gtc agc ctg acc tgc ctg gtc      864
   Ser Arg Asp Glu Leu Thr Lys Asn Gln Val Ser Leu Thr Cys Leu Val
           275                 280                 285
   aaa ggc ttc tat ccc agc gac atc gcc gtg gag tgg gag agc aat ggg      912
   Lys Gly Phe Tyr Pro Ser Asp Ile Ala Val Glu Trp Glu Ser Asn Gly
           290                 295                 300
   cag ccg gag aac aac tac aag acc acg cct ccc gtg ttg gac tcc gac       960
   Gln Pro Glu Asn Asn Tyr Lys Thr Thr Pro Pro Val Leu Asp Ser Asp
   305                 310                 315                 320
   ggc tcc ttc ttc ctc tac agc aag ctc acc gtg gac aag agc agg tgg      1008
   Gly Ser Phe Phe Leu Tyr Ser Lys Leu Thr Val Asp Lys Ser Arg Trp
                   325                 330                 335
   cag cag ggg aac gtc ttc tca tgc tcc gtg atg cat gag gct ctg cac      1056
   Gln Gln Gly Asn Val Phe Ser Cys Ser Val Met His Glu Ala Leu His
               340                 345                 350
   aac cac tac acg cag aag agc ctc tcc ctg tct ccc ggg aaa tga          1101
   Asn His Tyr Thr Gln Lys Ser Leu Ser Leu Ser Pro Gly Lys
           355                 360                 365
<210> 6
   <211> 366
   <212> PRT
   <213> Homo sapiens
<400> 6
   Met Glu Thr Asp Thr Leu Leu Leu Trp Val Leu Leu Leu Trp Val Pro
   1               5                   10                  15
   Gly Ser Thr Gly Asp Val Thr Met Ser Gly Leu Gly Arg Ser Arg Arg
               20                  25                  30
   Gly Gly Arg Ser Arg Val Asp Gln Glu Glu Arg Phe Pro Gln Gly Leu
           35                  40                  45
   Trp Thr Gly Val Ala Met Arg Ser Cys Pro Glu Glu Gln Tyr Trp Asp
       50                  55                  60
   Pro Leu Leu Gly Thr Cys Met Ser Cys Lys Thr Ile Cys Asn His Gln
   65                  70                  75                  80
   Ser Gln Arg Thr Cys Ala Ala Phe Cys Arg Ser Leu Ser Cys Arg Lys
                   85                  90                  95
   Glu Gln Gly Lys Phe Tyr Asp His Leu Leu Arg Asp Cys Ile Ser Cys
               100                 105                 110
   Ala Ser Ile Cys Gly Gln His Pro Lys Gln Cys Ala Tyr Phe Cys Glu
           115                 120                 125
   Asn Lys Leu Arg Ser Pro Val Asn Leu Pro Val Asp Lys Thr His Thr
       130                 135                 140
   Cys Pro Pro Cys Pro Ala Pro Glu Leu Leu Gly Gly Pro Ser Val Phe
   145                 150                 155                 160
   Leu Phe Pro Pro Lys Pro Lys Asp Thr Leu Met Ile Ser Arg Thr Pro
                   165                 170                 175
   Glu Val Thr Cys Val Val Val Asp Val Ser His Glu Asp Pro Glu Val
               180                 185                 190
   Lys Phe Asn Trp Tyr Val Asp Gly Val Glu Val His Asn Ala Lys Thr
           195                 200                 205
   Lys Pro Arg Glu Glu Gln Tyr Asn Ser Thr Tyr Arg Val Val Ser Val
       210                 215                 220
   Leu Thr Val Leu His Gln Asp Trp Leu Asn Gly Lys Glu Tyr Lys Cys
   225                 230                 235                 240
   Lys Val Ser Asn Lys Ala Leu Pro Ala Pro Ile Glu Lys Thr Ile Ser
                   245                 250                 255
   Lys Ala Lys Gly Gln Pro Arg Glu Pro Gln Val Tyr Thr Leu Pro Pro
               260                 265                 270
   Ser Arg Asp Glu Leu Thr Lys Asn Gln Val Ser Leu Thr Cys Leu Val
           275                 280                 285
   Lys Gly Phe Tyr Pro Ser Asp Ile Ala Val Glu Trp Glu Ser Asn Gly
       290                 295                 300
   Gln Pro Glu Asn Asn Tyr Lys Thr Thr Pro Pro Val Leu Asp Ser Asp
   305                 310                 315                 320
   Gly Ser Phe Phe Leu Tyr Ser Lys Leu Thr Val Asp Lys Ser Arg Trp
                   325                 330                 335
   Gln Gln Gly Asn Val Phe Ser Cys Ser Val Met His Glu Ala Leu His
               340                 345                 350
   Asn His Tyr Thr Gln Lys Ser Leu Ser Leu Ser Pro Gly Lys
           355                 360                 365
<210> 7
   <211> 1005
   <212> DNA
   <213> Homo sapiens
<220>
   <221> CDS
   <222> (1)..(1005)
   <223>
<400> 7
   atg gag aca gac aca ctc ctg tta tgg gtg ctg ctg ctc tgg gtt cca      48
   Met Glu Thr Asp Thr Leu Leu Leu Trp Val Leu Leu Leu Trp Val Pro
   1               5                   10                  15
   ggt tcc act ggt gac gtc aga tcc tgc ccc gaa gag cag tac tgg gat      96
   Gly Ser Thr Gly Asp Val Arg Ser Cys Pro Glu Glu Gln Tyr Trp Asp
               20                  25                  30
   cct ctg ctg ggt acc tgc atg tcc tgc aaa acc att tgc aac cat cag      144
   Pro Leu Leu Gly Thr Cys Met Ser Cys Lys Thr Ile Cys Asn His Gln
           35                  40                  45
   agc cag cgc acc tgt gca gcc ttc tgc agg tca ctc agc tgc cgc aag      192
   Ser Gln Arg Thr Cys Ala Ala Phe Cys Arg Ser Leu Ser Cys Arg Lys
       50                  55                  60
   gag caa ggc aag ttc tat gac cat ctc ctg agg gac tgc atc agc tgt      240
   Glu Gln Gly Lys Phe Tyr Asp His Leu Leu Arg Asp Cys Ile Ser Cys
   65                  70                  75                  80
   gcc tcc atc tgt gga cag cac cct aag caa tgt gca tac ttc tgt gag      288
   Ala Ser Ile Cys Gly Gln His Pro Lys Gln Cys Ala Tyr Phe Cys Glu
                   85                  90                  95
   aac aag ctc agg agc cca gtg aac ctt cca gtc gac aaa act cac aca      336
   Asn Lys Leu Arg Ser Pro Val Asn Leu Pro Val Asp Lys Thr His Thr
               100                 105                 110
   tgc cca ccg tgc cca gca cct gaa ctc ctg ggg gga ccg tca gtc ttc      384
   Cys Pro Pro Cys Pro Ala Pro Glu Leu Leu Gly Gly Pro Ser Val Phe
           115                 120                 125
   ctc ttc ccc cca aaa ccc aag gac acc ctc atg atc tcc cgg acc cct      432
   Leu Phe Pro Pro Lys Pro Lys Asp Thr Leu Met Ile Ser Arg Thr Pro
       130                 135                 140
   gag gtc aca tgc gtg gtg gtg gac gtg agc cac gaa gac cct gag gtc      480
   Glu Val Thr Cys Val Val Val Asp Val Ser His Glu Asp Pro Glu Val
   145                 150                 155                 160
   aag ttc aac tgg tac gtg gac ggc gtg gag gtg cat aat gcc aag aca      528
   Lys Phe Asn Trp Tyr Val Asp Gly Val Glu Val His Asn Ala Lys Thr
                   165                 170                 175
   aag ccg cgg gag gag cag tac aac agc acg tac cgt gtg gtc agc gtc      576
   Lys Pro Arg Glu Glu Gln Tyr Asn Ser Thr Tyr Arg Val Val Ser Val
               180                 185                 190
   ctc acc gtc ctg cac cag gac tgg ctg aat ggc aag gag tac aag tgc      624
   Leu Thr Val Leu His Gln Asp Trp Leu Asn Gly Lys Glu Tyr Lys Cys
           195                 200                 205
   aag gtc tcc aac aaa gcc ctc cca gcc ccc atc gag aaa acc atc tcc      672
   Lys Val Ser Asn Lys Ala Leu Pro Ala Pro Ile Glu Lys Thr Ile Ser
       210                 215                 220
   aaa gcc aaa ggg cag ccc cga gaa cca cag gtg tac acc ctg ccc cca      720
   Lys Ala Lys Gly Gln Pro Arg Glu Pro Gln Val Tyr Thr Leu Pro Pro
   225                 230                 235                 240
   tcc cgg gat gag ctg acc aag aac cag gtc agc ctg acc tgc ctg gtc      768
   Ser Arg Asp Glu Leu Thr Lys Asn Gln Val Ser Leu Thr Cys Leu Val
                   245                 250                 255
   aaa ggc ttc tat ccc agc gac atc gcc gtg gag tgg gag agc aat ggg      816
   Lys Gly Phe Tyr Pro Ser Asp Ile Ala Val Glu Trp Glu Ser Asn Gly
               260                 265                 270
   cag ccg gag aac aac tac aag acc acg cct ccc gtg ttg gac tcc gac        864
   Gln Pro Glu Asn Asn Tyr Lys Thr Thr Pro Pro Val Leu Asp Ser Asp
           275                 280                 285
   ggc tcc ttc ttc ctc tac agc aag ctc acc gtg gac aag agc agg tgg        912
   Gly Ser Phe Phe Leu Tyr Ser Lys Leu Thr Val Asp Lys Ser Arg Trp
       290                 295                 300
   cag cag ggg aac gtc ttc tca tgc tcc gtg atg cat gag gct ctg cac        960
   Gln Gln Gly Asn Val Phe Ser Cys Ser Val Met His Glu Ala Leu His
   305                 310                 315                 320
   aac cac tac acg cag aag agc ctc tcc ctg tct ccc ggg aaa tga           1005
   Asn His Tyr Thr Gln Lys Ser Leu Ser Leu Ser Pro Gly Lys
                   325                 330
<210> 8
   <211> 334
   <212> PRT
   <213> Homo sapiens
<400> 8
   Met Glu Thr Asp Thr Leu Leu Leu Trp Val Leu Leu Leu Trp Val Pro
   1               5                   10                  15
   Gly Ser Thr Gly Asp Val Arg Ser Cys Pro Glu Glu Gln Tyr Trp Asp
               20                  25                  30
   Pro Leu Leu Gly Thr Cys Met Ser Cys Lys Thr Ile Cys Asn His Gln
           35                  40                  45
   Ser Gln Arg Thr Cys Ala Ala Phe Cys Arg Ser Leu Ser Cys Arg Lys
       50                  55                  60
   Glu Gln Gly Lys Phe Tyr Asp His Leu Leu Arg Asp Cys Ile Ser Cys
   65                  70                  75                  80
   Ala Ser Ile Cys Gly Gln His Pro Lys Gln Cys Ala Tyr Phe Cys Glu
                   85                  90                  95
   Asn Lys Leu Arg Ser Pro Val Asn Leu Pro Val Asp Lys Thr His Thr
               100                 105                 110
   Cys Pro Pro Cys Pro Ala Pro Glu Leu Leu Gly Gly Pro Ser Val Phe
           115                 120                 125
   Leu Phe Pro Pro Lys Pro Lys Asp Thr Leu Met Ile Ser Arg Thr Pro
       130                 135                 140
   Glu Val Thr Cys Val Val Val Asp Val Ser His Glu Asp Pro Glu Val
   145                 150                 155                 160
   Lys Phe Asn Trp Tyr Val Asp Gly Val Glu Val His Asn Ala Lys Thr
                   165                 170                 175
   Lys Pro Arg Glu Glu Gln Tyr Asn Ser Thr Tyr Arg Val Val Ser Val
               180                 185                 190
   Leu Thr Val Leu His Gln Asp Trp Leu Asn Gly Lys Glu Tyr Lys Cys
           195                 200                 205
   Lys Val Ser Asn Lys Ala Leu Pro Ala Pro Ile Glu Lys Thr Ile Ser
       210                 215                 220
   Lys Ala Lys Gly Gln Pro Arg Glu Pro Gln Val Tyr Thr Leu Pro Pro
   225                 230                 235                 240
   Ser Arg Asp Glu Leu Thr Lys Asn Gln Val Ser Leu Thr Cys Leu Val
                   245                 250                 255
   Lys Gly Phe Tyr Pro Ser Asp Ile Ala Val Glu Trp Glu Ser Asn Gly
               260                 265                 270
   Gln Pro Glu Asn Asn Tyr Lys Thr Thr Pro Pro Val Leu Asp Ser Asp
           275                 280                 285
   Gly Ser Phe Phe Leu Tyr Ser Lys Leu Thr Val Asp Lys Ser Arg Trp
       290                 295                 300
   Gln Gln Gly Asn Val Phe Ser Cys Ser Val Met His Glu Ala Leu His
   305                 310                 315                 320
   Asn His Tyr Thr Gln Lys Ser Leu Ser Leu Ser Pro Gly Lys
                   325                 330
<210> 9
   <211> 531
   <212> DNA
   <213> Homo sapiens
<220>
   <221> CDS
   <222> (1)..(531)
   <223>
<400> 9
   atg gct atc atc tac ctc atc ctc ctg ttc acc gct gtg cgg ggc gat      48
   Met Ala Ile Ile Tyr Leu Ile Leu Leu Phe Thr Ala Val Arg Gly Asp
   1               5                   10                  15
   tac aaa gac gat gac gat aaa gga ccc gga cag gtg cag ctg cag gca      96
   Tyr Lys Asp Asp Asp Asp Lys Gly Pro Gly Gln Val Gln Leu Gln Ala
               20                  25                  30
   gtg ctc acc caa aaa cag aag aag cag cac tct gtc ctg cac ctg gtt     144
   Val Leu Thr Gln Lys Gln Lys Lys Gln His Ser Val Leu His Leu Val
           35                  40                  45
   ccc att aac gcc acc tcc aag gat gac tcc gat gtg aca gag gtg atg      192
   Pro Ile Asn Ala Thr Ser Lys Asp Asp Ser Asp Val Thr Glu Val Met
       50                  55                  60
   tgg caa cca gct ctt agg cgt ggg aga ggc cta cag gcc caa gga tat      240
   Trp Gln Pro Ala Leu Arg Arg Gly Arg Gly Leu Gln Ala Gln Gly Tyr
   65                  70                  75                  80
   ggt gtc cga atc cag gat gct gga gtt tat ctg ctg tat agc cag gtc      288
   Gly Val Arg Ile Gln Asp Ala Gly Val Tyr Leu Leu Tyr Ser Gln Val
                   85                  90                  95
   ctg ttt caa gac gtg act ttc acc atg ggt cag gtg gtg tct cga gaa      336
   Leu Phe Gln Asp Val Thr Phe Thr Met Gly Gln Val Val Ser Arg Glu
               100                 105                 110
   ggc caa gga agg cag gag act cta ttc cga tgt ata aga agt atg ccc      384
   Gly Gln Gly Arg Gln Glu Thr Leu Phe Arg Cys Ile Arg Ser Met Pro
           115                 120                 125
   tcc cac ccg gac cgg gcc tac aac agc tgc tat agc gca ggt gtc ttc      432
   Ser His Pro Asp Arg Ala Tyr Asn Ser Cys Tyr Ser Ala Gly Val Phe
       130                 135                 140
   cat tta cac caa ggg gat att ctg agt gtc ata att ccc cgg gca agg      480
   His Leu His Gln Gly Asp Ile Leu Ser Val Ile Ile Pro Arg Ala Arg
   145                 150                 155                 160
   gcg aaa ctt aac ctc tct cca cat gga acc ttc ctg ggg ttt gtg aaa      528
   Ala Lys Leu Asn Leu Ser Pro His Gly Thr Phe Leu Gly Phe Val Lys
                   165                 170                 175
   ctg                                                                  531
   Leu
<210> 10
   <211> 177
   <212> PRT
   <213> Homo sapiens
<400> 10
   Met Ala Ile Ile Tyr Leu Ile Leu Leu Phe Thr Ala Val Arg Gly Asp
   1               5                   10                  15
   Tyr Lys Asp Asp Asp Asp Lys Gly Pro Gly Gln Val Gln Leu Gln Ala
               20                  25                  30
   Val Leu Thr Gln Lys Gln Lys Lys Gln His Ser Val Leu His Leu Val
           35                  40                  45
   Pro Ile Asn Ala Thr Ser Lys Asp Asp Ser Asp Val Thr Glu Val Met
       50                  55                  60
   Trp Gln Pro Ala Leu Arg Arg Gly Arg Gly Leu Gln Ala Gln Gly Tyr
   65                  70                  75                  80
   Gly Val Arg Ile Gln Asp Ala Gly Val Tyr Leu Leu Tyr Ser Gln Val
                   85                  90                  95
   Leu Phe Gln Asp Val Thr Phe Thr Met Gly Gln Val Val Ser Arg Glu
               100                 105                 110
   Gly Gln Gly Arg Gln Glu Thr Leu Phe Arg Cys Ile Arg Ser Met Pro
           115                 120                 125
   Ser His Pro Asp Arg Ala Tyr Asn Ser Cys Tyr Ser Ala Gly Val Phe
       130                 135                 140
   His Leu His Gln Gly Asp Ile Leu Ser Val Ile Ile Pro Arg Ala Arg
   145                 150                 155                 160
   Ala Lys Leu Asn Leu Ser Pro His Gly Thr Phe Leu Gly Phe Val Lys
                   165                 170                 175
   Leu
<210> 11
   <211> 1470
   <212> DNA
   <213> Homo sapiens
<220>
   <221> CDS
   <222> (119)..(1021)
   <223>
<220>
   <221> CDS
   <222> (1025)..(1054)
   <223>
<400> 11
   acgcaaatgg gcggtaggcg tgtacggtgg gaggtctata taagcagagc tcgtttagtg      60
   aaccgtcaga tctctagaag ctgggtacca gctgctagca agcttgctag cggccgcc       118
   atg gac tac aaa gac gat gac gac aag agt ggc ctg ggc cgg agc agg       166
   Met Asp Tyr Lys Asp Asp Asp Asp Lys Ser Gly Leu Gly Arg Ser Arg
   1               5                   10                  15
   cga ggt ggc cgg agc cgt gtg gac cag gag gag cgc ttt cca cag ggc       214
   Arg Gly Gly Arg Ser Arg Val Asp Gln Glu Glu Arg Phe Pro Gln Gly
               20                  25                  30
   ctg tgg acg ggg gtg gct atg aga tcc tgc ccc gaa gag cag tac tgg       262
   Leu Trp Thr Gly Val Ala Met Arg Ser Cys Pro Glu Glu Gln Tyr Trp
           35                  40                  45
   gat cct ctg ctg ggt acc tgc atg tcc tgc aaa acc att tgc aac cat       310
   Asp Pro Leu Leu Gly Thr Cys Met Ser Cys Lys Thr Ile Cys Asn His
       50                  55                  60
   cag agc cag cgc acc tgt gca gcc ttc tgc agg tca ctc agc tgc cgc       358
   Gln Ser Gln Arg Thr Cys Ala Ala Phe Cys Arg Ser Leu Ser Cys Arg
   65                  70                  75                  80
   aag gag caa ggc aag ttc tat gac cat ctc ctg agg gac tgc atc agc      406
   Lys Glu Gln Gly Lys Phe Tyr Asp His Leu Leu Arg Asp Cys Ile Ser
                   85                  90                  95
   tgt gcc tcc atc tgt gga cag cac cct aag caa tgt gca tac ttc tgt      454
   Cys Ala Ser Ile Cys Gly Gln His Pro Lys Gln Cys Ala Tyr Phe Cys
               100                 105                 110
   gag aac aag ctc agg agc cca gtg aac ctt cca cca gag ctc agg aga      502
   Glu Asn Lys Leu Arg Ser Pro Val Asn Leu Pro Pro Glu Leu Arg Arg
           115                 120                 125
   cag cgg agt gga gaa gtt gaa aac aat tca gac aac tcg gga agg tac      550
   Gln Arg Ser Gly Glu Val Glu Asn Asn Ser Asp Asn Ser Gly Arg Tyr
       130                 135                 140
   caa gga ttg gag cac aga ggc tca gaa gca agt cca gct ctc ccg ggg      598
   Gln Gly Leu Glu His Arg Gly Ser Glu Ala Ser Pro Ala Leu Pro Gly
   145                 150                 155                 160
   ctg aag ctg agt gca gat cag gtg gcc ctg gtc tac agc acg ctg ggg      646
   Leu Lys Leu Ser Ala Asp Gln Val Ala Leu Val Tyr Ser Thr Leu Gly
                   165                 170                 175
   ctc tgc ctg tgt gcc gtc ctc tgc tgc ttc ctg gtg gcg gtg gcc tgc      694
   Leu Cys Leu Cys Ala Val Leu Cys Cys Phe Leu Val Ala Val Ala Cys
               180                 185                 190
   ttc ctc aag aag agg ggg gat ccc tgc tcc tgc cag ccc cgc tca agg      742
   Phe Leu Lys Lys Arg Gly Asp Pro Cys Ser Cys Gln Pro Arg Ser Arg
           195                 200                 205
   ccc cgt caa agt ccg gcc aag tct tcc cag gat cac gcg atg gaa gcc      790
   Pro Arg Gln Ser Pro Ala Lys Ser Ser Gln Asp His Ala Met Glu Ala
       210                 215                 220
   ggc agc cct gtg agc aca tcc ccc gag cca gtg gag acc tgc agc ttc      838
   Gly Ser Pro Val Ser Thr Ser Pro Glu Pro Val Glu Thr Cys Ser Phe
   225                 230                 235                 240
   tgc ttc cct gag tgc agg gcg ccc acg cag gag agc gca gtc acg cct      886
   Cys Phe Pro Glu Cys Arg Ala Pro Thr Gln Glu Ser Ala Val Thr Pro
                   245                 250                 255
   ggg acc ccc gac ccc act tgt gct gga agg tgg ggg tgc cac acc agg      934
   Gly Thr Pro Asp Pro Thr Cys Ala Gly Arg Trp Gly Cys His Thr Arg
               260                 265                 270
   acc aca gtc ctg cag cct tgc cca cac atc cca gac agt ggc ctt ggc      982
   Thr Thr Val Leu Gln Pro Cys Pro His Ile Pro Asp Ser Gly Leu Gly
           275                 280                 285
   att gtg tgt gtg cct gcc cag gag ggg ggc cca ggt gca taa atg ggg     1030
   Ile Val Cys Val Pro Ala Gln Glu Gly Gly Pro Gly Ala     Met Gly
       290                 295                 300
   gtc agc ggc cgc tcg agg ccg gca aggccggatc cagacatgat aagatacatt    1084
   Val Ser Gly Arg Ser Arg Pro Ala
       305                 310
   gatgagtttg gacaaaccac aactagaatg cagtgaaaaa aatgctttat ttgtgaaatt   1144
   tgtgatgcta ttgctttatt tgtaaccatt ataagctgca ataaacaagt taacaacaac   1204
   aattgcattc attttatgtt tcaggttcag ggggaggtgg ggaggttttt taaagcaagt 1264
   aaaacctcta caaatgtggt atggctgatt atgatccggc tgcctcgcgc gtttcggtga 1324
   tgacggtgaa aacctctgac acatgcagct cccggagacg gtcacagctt gtctgtaagc 1384
   ggatgccggg agcagacaag cccgtcaggg cgcgtcagcg ggtgttggcg ggtgtcgggg 1444
   cgcagccatg accggtcgac tctaga 1470
<210> 12
   <211> 301
   <212> PRT
   <213> Homo sapiens
<400> 12
   Met Asp Tyr Lys Asp Asp Asp Asp Lys Ser Gly Leu Gly Arg Ser Arg
   1 5 10 15
   Arg Gly Gly Arg Ser Arg Val Asp Gln Glu Glu Arg Phe Pro Gln Gly
   20 25 30
   Leu Trp Thr Gly Val Ala Met Arg Ser Cys Pro Glu Glu Gln Tyr Trp
   35 40 45
   Asp Pro Leu Leu Gly Thr Cys Met Ser Cys Lys Thr Ile Cys Asn His
   50 55 60
   Gln Ser Gln Arg Thr Cys Ala Ala Phe Cys Arg Ser Leu Ser Cys Arg
   65 70 75 80
   Lys Glu Gln Gly Lys Phe Tyr Asp His Leu Leu Arg Asp Cys Ile Ser
   85 90 95
   Cys Ala Ser Ile Cys Gly Gln His Pro Lys Gln Cys Ala Tyr Phe Cys
   100 105 110
   Glu Asn Lys Leu Arg Ser Pro Val Asn Leu Pro Pro Glu Leu Arg Arg
   115 120 125
   Gln Arg Ser Gly Glu Val Glu Asn Asn Ser Asp Asn Ser Gly Arg Tyr
   130 135 140
   Gln Gly Leu Glu His Arg Gly Ser Glu Ala Ser Pro Ala Leu Pro Gly
   145 150 155 160
   Leu Lys Leu Ser Ala Asp Gln Val Ala Leu Val Tyr Ser Thr Leu Gly
   165 170 175
   Leu Cys Leu Cys Ala Val Leu Cys Cys Phe Leu Val Ala Val Ala Cys
   180 185 190
   Phe Leu Lys Lys Arg Gly Asp Pro Cys Ser Cys Gln Pro Arg Ser Arg
   195 200 205
   Pro Arg Gln Ser Pro Ala Lys Ser Ser Gln Asp His Ala Met Glu Ala
   210 215 220
   Gly Ser Pro Val Ser Thr Ser Pro Glu Pro Val Glu Thr Cys Ser Phe
   225 230 235 240
   Cys Phe Pro Glu Cys Arg Ala Pro Thr Gln Glu Ser Ala Val Thr Pro
   245 250 255
   Gly Thr Pro Asp Pro Thr Cys Ala Gly Arg Trp Gly Cys His Thr Arg
   260 265 270
   Thr Thr Val Leu Gln Pro Cys Pro His Ile Pro Asp Ser Gly Leu Gly
   275 280 285
   Ile Val Cys Val Pro Ala Gln Glu Gly Gly Pro Gly Ala
   290 295 300
<210> 13
   <211> 10
   <212> PRT
   <213> Homo sapiens
<400> 13
   Met Gly Val Ser Gly Arg Ser Arg Pro Ala
   1 5 10
<210> 14
   <211> 1304
   <212> DNA
   <213> Homo sapiens
<220>
   <221> CDS
   <222> (42)..(1253)
   <223>
<400> 14
   taatacgact cactataggg agacccaagc ttaatcaaaa c atg gct atc atc tac      56
   ctc atc ctc ctg ttc acc gct gtg cgg ggc ctc gac atg agt ggc ctg      104
   ggc cgg agc agg cga ggt ggc cgg agc cgt gtg gac cag gag gag cgc      152
   ttt cca cag ggc ctg tgg acg ggg gtg gct atg aga tcc tgc ccc gaa      200
   gag cag tac tgg gat cct ctg ctg ggt acc tgc atg tcc tgc aaa acc      248
   att tgc aac cat cag agc cag cgc acc tgt gca gcc ttc tgc agg tca      296
   ctc agc tgc cgc aag gag caa ggc aag ttc tat gac cat ctc ctg agg      344
   gac tgc atc agc tgt gcc tcc atc tgt gga cag cac cct aag caa tgt      392
   gca tac ttc tgt gag aac aag ctc agg agc cca gtg aac ctt cca cca      440
   gag ctc agg aga cag cgg agt gga gaa gtt gaa aac aat tca gac aac      488
   tcg gga agg tac caa gga ttg gag cac aga ggc tca gaa gca agt cca      536
   gct ctc ccg ggg ctg aag ctg agt gca gat cag gtc gac aaa act cac      584
   aca tgc cca ccg tgc cca gca cct gaa ctc ctg ggg gga ccg tca gtc      632
   ttc ctc ttc ccc cca aaa ccc aag gac acc ctc atg atc tcc cgg acc      680
   cct gag gtc aca tgc gtg gtg gtg gac gtg agc cac gaa gac cct gag      728
   gtc aag ttc aac tgg tac gtg gac ggc gtg gag gtg cat aat gcc aag      776
   aca aag ccg cgg gag gag cag tac aac agc acg tac cgt gtg gtc agc      824
   gtc ctc acc gtc ctg cac cag gac tgg ctg aat ggc aag gag tac aag      872
   tgc aag gtc tcc aac aaa gcc ctc cca gcc ccc atc gag aaa acc atc      920
   tcc aaa gcc aaa ggg cag ccc cga gaa cca cag gtg tac acc ctg ccc      968
   cca tcc cgg gat gag ctg acc aag aac cag gtc agc ctg acc tgc ctg     1016
   gtc aaa ggc ttc tat ccc agc gac atc gcc gtg gag tgg gag agc aat      1064
   ggg cag ccg gag aac aac tac aag acc acg cct ccc gtg ttg gac tcc      1112
   gac ggc tcc ttc ttc ctc tac agc aag ctc acc gtg gac aag agc agg      1160
   tgg cag cag ggg aac gtc ttc tca tgc tcc gtg atg cat gag gct ctg      1208
   cac aac cac tac acg cag aag agc ctc tcc ctg tct ccg ggt aaa          1253
   tgagtgcgcg cggccgctct agagggccct attctatagt gtcacctaaa t             1304
<210> 15
   <211> 404
   <212> PRT
   <213> Homo sapiens
<400> 15
<210> 16
   <211> 546
   <212> DNA
   <213> Mus musculus
<220>
   <221> CDS
   <222> (16)..(543)
   <223>
<400> 16
   aagcttaatc aaaac atg gct atc atc tac ctc atc ctc ctg ttc acc gct      51
                    Met Ala Ile Ile Tyr Leu Ile Leu Leu Phe Thr Ala
                    1               5                   10
   gtg cgg ggc gat tac aaa gac gat gac gat aaa gga ccc gga cag gtg       99
   Val Arg Gly Asp Tyr Lys Asp Asp Asp Asp Lys Gly Pro Gly Gln Val
           15                  20                  25
   cag ctg cag gca gta ctc acc cag aag cac aag aag aag cac tca gtc      147
   Gln Leu Gln Ala Val Leu Thr Gln Lys His Lys Lys Lys His Ser Val
       30                  35                  40
   ctg cat ctt gtt cca gtt aac att acc tcc aag gac tct gac gtg aca      195
   Leu His Leu Val Pro Val Asn Ile Thr Ser Lys Asp Ser Asp Val Thr
   45                  50                  55                  60
   gag gtg atg tgg caa cca gta ctt agg cgt ggg aga ggc ctg gag gcc      243
   Glu Val Met Trp Gln Pro Val Leu Arg Arg Gly Arg Gly Leu Glu Ala
                   65                  70                  75
   cag gga gac att gta cga gtc tgg gac act gga att tat ctg ctc tat      291
   Gln Gly Asp Ile Val Arg Val Trp Asp Thr Gly Ile Tyr Leu Leu Tyr
               80                  85                  90
   agt cag gtc ctg ttt cat gat gtg act ttc aca atg ggt cag gtg gta      339
   Ser Gln Val Leu Phe His Asp Val Thr Phe Thr Met Gly Gln Val Val
           95                  100                 105
   tct cgg gaa gga caa ggg aga aga gaa act cta ttc cga tgt atc aga      387
   Ser Arg Glu Gly Gln Gly Arg Arg Glu Thr Leu Phe Arg Cys Ile Arg
       110                 115                 120
   agt atg cct tct gat cct gac cgt gcc tac aat agc tgc tac agt gca      435
   Ser Met Pro Ser Asp Pro Asp Arg Ala Tyr Asn Ser Cys Tyr Ser Ala
   125                 130                 135                 140
   ggt gtc ttt cat tta cat caa ggg gat att atc act gtc aaa att cca      483
   Gly Val Phe His Leu His Gln Gly Asp Ile Ile Thr Val Lys Ile Pro
                   145                 150                 155
   cgg gca aac gca aaa ctt agc ctt tct ccg cat gga aca ttc ctg ggg      531
   Arg Ala Asn Ala Lys Leu Ser Leu Ser Pro His Gly Thr Phe Leu Gly
               160                 165                 170
   ttt gtg aaa cta tga                                                  546
   Phe Val Lys Leu
           175
<210> 17
   <211> 176
   <212> PRT
   <213> Mus musculus
<400> 17
   Met Ala Ile Ile Tyr Leu Ile Leu Leu Phe Thr Ala Val Arg Gly Asp
   1               5                   10                  15
   Tyr Lys Asp Asp Asp Asp Lys Gly Pro Gly Gln Val Gln Leu Gln Ala
               20                  25                  30
   Val Leu Thr Gln Lys His Lys Lys Lys His Ser Val Leu His Leu Val
           35                  40                  45
   Pro Val Asn Ile Thr Ser Lys Asp Ser Asp Val Thr Glu Val Met Trp
       50                  55                  60
   Gln Pro Val Leu Arg Arg Gly Arg Gly Leu Glu Ala Gln Gly Asp Ile
   65                  70                  75                  80
   Val Arg Val Trp Asp Thr Gly Ile Tyr Leu Leu Tyr Ser Gln Val Leu
                   85                  90                  95
   Phe His Asp Val Thr Phe Thr Met Gly Gln Val Val Ser Arg Glu Gly
               100                 105                 110
   Gln Gly Arg Arg Glu Thr Leu Phe Arg Cys Ile Arg Ser Met Pro Ser
           115                 120                 125
   Asp Pro Asp Arg Ala Tyr Asn Ser Cys Tyr Ser Ala Gly Val Phe His
       130                 135                 140
   Leu His Gln Gly Asp Ile Ile Thr Val Lys Ile Pro Arg Ala Asn Ala
   145                 150                 155                 160
   Lys Leu Ser Leu Ser Pro His Gly Thr Phe Leu Gly Phe Val Lys Leu
                   165                 170                 175

### SEQUENCE LISTING

<110> Biogen, Inc.
<120> Taci As As Anti-Tumor Agent
<130> P031883EP
<150> USSN 60/199,946
   <151> 2000-04-27
<160> 17
<170> PatentIn version 3.1
<210> 1
   <211> 293
   <212> PRT
   <213> Homo sapiens
<400> 1
<210> 2
   <211> 882
   <212> DNA
   <213> Homo sapiens
<400> 2
   atgagtggcc tgggccggag caggcgaggt ggccggagcc gtgtggacca ggaggagcgc      60
   tttccacagg gcctgtggac gggggtggct atgagatcct gccccgaaga gcagtactgg     120
   gatcctctgc tgggtacctg catgtcctgc aaaaccattt gcaaccatca gagccagcgc     180
   acctgtgcag ccttctgcag gtcactcagc tgccgcaagg agcaaggcaa gttctatgac     240
   catctcctga gggactgcat cagctgtgcc tccatctgtg gacagcaccc taagcaatgt     300
   gcatacttct gtgagaacaa gctcaggagc ccagtgaacc ttccaccaga gctcaggaga     360
   cagcggagtg gagaagttga aaacaattca gacaactcgg gaaggtacca aggattggag     420
   cacagaggct cagaagcaag tccagctctc ccggggctga agctgagtgc agatcaggtg     480
   gccctggtct acagcacgct ggggctctgc ctgtgtgccg tcctctgctg cttcctggtg     540
   gcggtggcct gcttcctcaa gaagaggggg gatccctgct cctgccagcc ccgctcaagg     600
   ccccgtcaaa gtccggccaa gtcttcccag gatcacgcga tggaagccgg cagccctgtg     660
   agcacatccc ccgagccagt ggagacctgc agcttctgct tccctgagtg cagggcgccc     720
   acgcaggaga gcgcagtcac gcctgggacc cccgacccca cttgtgctgg aaggtggggg     780
   tgccacacca ggaccacagt cctgcagcct tgcccacaca tcccagacag tggccttggc     840
   attgtgtgtg tgcctgccca ggaggggggc ccaggtgcat aa                        882
<210> 3
   <211> 249
   <212> PRT
   <213> Mus musculus
<400> 3
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Asp Glu Thr Ala Gln
               20                  25                  30
   Ile Pro Ala Glu Ala Val Ile Gly Tyr Ser Asp Leu Glu Gly Asp Phe
           35                  40                  45
   Asp Val Ala Val Leu Pro Phe Ser Asn Ser Thr Asn Asn Gly Leu Leu
       50                  55                  60
   Phe Ile Asn Thr Thr Ile Ala Ser Ile Ala Ala Lys Glu Glu Gly Val
   65                  70                  75                  80
   Ser Leu Glu Lys Arg Glu Gln Lys Leu Ile Ser Glu Glu Asp Leu Asn
                   85                  90                  95
   Gly Gly Gly Gly Ser Lys Glu Leu Ala Val Leu Thr Gln Lys His Lys
               100                 105                 110
   Lys Lys His Ser Val Leu His Leu Val Pro Val Asn Ile Thr Ser Lys
   115 120 125
   Asp Ser Asp Val Thr Glu Val Met Trp Gln Pro Val Leu Arg Arg Gly
   130 135 140
   Arg Gly Leu Glu Ala Gln Gly Asp Ile Val Arg Val Trp Asp Thr Gly
   145 150 155 160
   Ile Tyr Leu Leu Tyr Ser Gln Val Leu Phe His Asp Val Thr Phe Thr
   165 170 175
   Met Gly Gln Val Val Ser Arg Glu Gly Gln Gly Arg Arg Glu Thr Leu
   180 185 190
   Phe Arg Cys Ile Arg Ser Met Pro Ser Asp Pro Asp Arg Ala Tyr Asn
   195 200 205
   Ser Cys Tyr Ser Ala Gly Val Phe His Leu His Gln Gly Asp Ile Ile
   210 215 220
   Thr Val Lys Ile Pro Arg Ala Asn Ala Lys Leu Ser Leu Ser Pro His
   225 230 235 240
   Gly Thr Phe Leu Gly Phe Val Lys Leu
   245
<210> 4
   <211> 788
   <212> DNA
   <213> Mus musculus
<400> 4
   gatccaaacg atgagatttc cttcaatttt tactgcagtt ttattcgcag catcctccgc 60
   attagctgct ccagtcaaca ctacaacaga agatgaaacg gcacaaattc cggctgaagc 120
   tgtcatcggt tactcagatt tagaagggga tttcgatgtt gctgttttgc cattttccaa 180
   cagcacaaat aacgggttat tgtttataaa tactactatt gccagcattg ctgctaaaga 240
   agaaggggta tctctcgaga aaagagaaca aaaactcatt tctgaggaag atctgaatgg 300
   tggcggtggg tccaaagagc tcgcagtact cacccagaag cacaagaaga agcactcagt 360
   cctgcatctt gttccagtta acattacctc caaggactct gacgtgacag aggtgatgtg 420
   gcaaccagta cttaggcgtg ggagaggcct ggaggcccag ggagacattg tacgagtctg 480
   ggacactgga atttatctgc tctatagtca ggtcctgttt catgatgtga ctttcacaat     540
   gggtcaggtg gtatctcggg aaggacaagg gagaagagaa actctattcc gatgtatcag     600
   aagtatgcct tctgatcctg accgtgccta caatagctgc tacagtgcag gtgtctttca     660
   tttacatcaa ggggatatta tcactgtcaa aattccacgg gcaaacgcaa aacttagcct     720
   ttctccgcat ggaacattcc tggggtttgt gaaactatga gcggccgcga attaattcgc     780
   cttagaca                                                              788
<210> 5
   <211> 1101
   <212> DNA
   <213> Homo sapiens
<220>
   <221> CDS
   <222> (1)..(1101)
   <223>
<400> 5
   atg gag aca gac aca ctc ctg tta tgg gtg ctg ctg ctc tgg gtt cca        48
   Met Glu Thr Asp Thr Leu Leu Leu Trp Val Leu Leu Leu Trp Val Pro
   1               5                   10                  15
   ggt tcc act ggt gac gtc acg atg agt ggc ctg ggc cgg agc agg cga        96
   Gly Ser Thr Gly Asp Val Thr Met Ser Gly Leu Gly Arg Ser Arg Arg
               20                  25                  30
   ggt ggc cgg agc cgt gtg gac cag gag gag cgc ttt cca cag ggc ctg       144
   Gly Gly Arg Ser Arg Val Asp Gln Glu Glu Arg Phe Pro Gln Gly Leu
           35                  40                  45
   tgg aca ggg gtg gct atg aga tcc tgc ccc gaa gag cag tac tgg gat       192
   Trp Thr Gly Val Ala Met Arg Ser Cys Pro Glu Glu Gln Tyr Trp Asp
       50                  55                  60
   cct ctg ctg ggt acc tgc atg tcc tgc aaa acc att tgc aac cat cag       240
   Pro Leu Leu Gly Thr Cys Met Ser Cys Lys Thr Ile Cys Asn His Gln
   65                  70                  75                  80
   agc cag cgc acc tgt gca gcc ttc tgc agg tca ctc agc tgc cgc aag       288
   Ser Gln Arg Thr Cys Ala Ala Phe Cys Arg Ser Leu Ser Cys Arg Lys
                   85                  90                  95
   gag caa ggc aag ttc tat gac cat ctc ctg agg gac tgc atc agc tgt       336
   Glu Gln Gly Lys Phe Tyr Asp His Leu Leu Arg Asp Cys Ile Ser Cys
               100                 105                 110
   gcc tcc atc tgt gga cag cac cct aag caa tgt gca tac ttc tgt gag       384
   Ala Ser Ile Cys Gly Gln His Pro Lys Gln Cys Ala Tyr Phe Cys Glu
           115                 120                 125
   aac aag ctc agg agc cca gtg aac ctt cca gtc gac aaa act cac aca        432
   Asn Lys Leu Arg Ser Pro Val Asn Leu Pro Val Asp Lys Thr His Thr
       130                 135                 140
   tgc cca ccg tgc cca gca cct gaa ctc ctg ggg gga ccg tca gtc ttc        480
   Cys Pro Pro Cys Pro Ala Pro Glu Leu Leu Gly Gly Pro Ser Val Phe
   145                 150                 155                 160
   ctc ttc ccc cca aaa ccc aag gac acc ctc atg atc tcc cgg acc cct        528
   Leu Phe Pro Pro Lys Pro Lys Asp Thr Leu Met Ile Ser Arg Thr Pro
                   165                 170                 175
   gag gtc aca tgc gtg gtg gtg gac gtg agc cac gaa gac cct gag gtc        576
   Glu Val Thr Cys Val Val Val Asp Val Ser His Glu Asp Pro Glu Val
               180                 185                 190
   aag ttc aac tgg tac gtg gac ggc gtg gag gtg cat aat gcc aag aca        624
   Lys Phe Asn Trp Tyr Val Asp Gly Val Glu Val His Asn Ala Lys Thr
           195                 200                 205
   aag ccg cgg gag gag cag tac aac agc acg tac cgt gtg gtc agc gtc        672
   Lys Pro Arg Glu Glu Gln Tyr Asn Ser Thr Tyr Arg Val Val Ser Val
       210                 215                 220
   ctc acc gtc ctg cac cag gac tgg ctg aat ggc aag gag tac aag tgc        720
   Leu Thr Val Leu His Gln Asp Trp Leu Asn Gly Lys Glu Tyr Lys Cys
   225                 230                 235                 240
   aag gtc tcc aac aaa gcc ctc cca gcc ccc atc gag aaa acc atc tcc        768
   Lys Val Ser Asn Lys Ala Leu Pro Ala Pro Ile Glu Lys Thr Ile Ser
                   245                 250                 255
   aaa gcc aaa ggg cag ccc cga gaa cca cag gtg tac acc ctg ccc cca        816
   Lys Ala Lys Gly Gln Pro Arg Glu Pro Gln Val Tyr Thr Leu Pro Pro
               260                 265                 270
   tcc cgg gat gag ctg acc aag aac cag gtc agc ctg acc tgc ctg gtc        864
   Ser Arg Asp Glu Leu Thr Lys Asn Gln Val Ser Leu Thr Cys Leu Val
           275                 280                 285
   aaa ggc ttc tat ccc agc gac atc gcc gtg gag tgg gag agc aat ggg        912
   Lys Gly Phe Tyr Pro Ser Asp Ile Ala Val Glu Trp Glu Ser Asn Gly
       290                 295                 300
   cag ccg gag aac aac tac aag acc acg cct ccc gtg ttg gac tcc gac        960
   Gln Pro Glu Asn Asn Tyr Lys Thr Thr Pro Pro Val Leu Asp Ser Asp
   305                 310                 315                 320
   ggc tcc ttc ttc ctc tac agc aag ctc acc gtg gac aag agc agg tgg       1008
   Gly Ser Phe Phe Leu Tyr Ser Lys Leu Thr Val Asp Lys Ser Arg Trp
                   325                 330                 335
   cag cag ggg aac gtc ttc tca tgc tcc gtg atg cat gag gct ctg cac       1056
   Gln Gln Gly Asn Val Phe Ser Cys Ser Val Met His Glu Ala Leu His
               340                 345                 350
   aac cac tac acg cag aag agc ctc tcc ctg tct ccc ggg aaa tga           1101
   Asn His Tyr Thr Gln Lys Ser Leu Ser Leu Ser Pro Gly Lys
   355 360 365
<210> 6
   <211> 366
   <212> PRT
   <213> Homo sapiens
<400> 6
   Met Glu Thr Asp Thr Leu Leu Leu Trp Val Leu Leu Leu Trp Val Pro
   1 5 10 15
   Gly Ser Thr Gly Asp Val Thr Met Ser Gly Leu Gly Arg Ser Arg Arg
   20 25 30
   Gly Gly Arg Ser Arg Val Asp Gln Glu Glu Arg Phe Pro Gln Gly Leu
   35 40 45
   Trp Thr Gly Val Ala Met Arg Ser Cys Pro Glu Glu Gln Tyr Trp Asp
   50 55 60
   Pro Leu Leu Gly Thr Cys Met Ser Cys Lys Thr Ile Cys Asn His Gln
   65 70 75 80
   Ser Gln Arg Thr Cys Ala Ala Phe Cys Arg Ser Leu Ser Cys Arg Lys
   85 90 95
   Glu Gln Gly Lys Phe Tyr Asp His Leu Leu Arg Asp Cys Ile Ser Cys
   100 105 110
   Ala Ser Ile Cys Gly Gln His Pro Lys Gln Cys Ala Tyr Phe Cys Glu
   115 120 125
   Asn Lys Leu Arg Ser Pro Val Asn Leu Pro Val Asp Lys Thr His Thr
   130 135 140
   Cys Pro Pro Cys Pro Ala Pro Glu Leu Leu Gly Gly Pro Ser Val Phe
   145 150 155 160
   Leu Phe Pro Pro Lys Pro Lys Asp Thr Leu Met Ile Ser Arg Thr Pro
   165 170 175
   Glu Val Thr Cys Val Val Val Asp Val Ser His Glu Asp Pro Glu Val
   180 185 190
   Lys Phe Asn Trp Tyr Val Asp Gly Val Glu Val His Asn Ala Lys Thr
           195                 200                 205
   Lys Pro Arg Glu Glu Gln Tyr Asn Ser Thr Tyr Arg Val Val Ser Val
       210                 215                 220
   Leu Thr Val Leu His Gln Asp Trp Leu Asn Gly Lys Glu Tyr Lys Cys
   225                 230                 235                 240
   Lys Val Ser Asn Lys Ala Leu Pro Ala Pro Ile Glu Lys Thr Ile Ser
                   245                 250                 255
   Lys Ala Lys Gly Gln Pro Arg Glu Pro Gln Val Tyr Thr Leu Pro Pro
               260                 265                 270
   Ser Arg Asp Glu Leu Thr Lys Asn Gln Val Ser Leu Thr Cys Leu Val
           275                 280                 285
   Lys Gly Phe Tyr Pro Ser Asp Ile Ala Val Glu Trp Glu Ser Asn Gly
       290                 295                 300
   Gln Pro Glu Asn Asn Tyr Lys Thr Thr Pro Pro Val Leu Asp Ser Asp
   305                 310                 315                 320
   Gly Ser Phe Phe Leu Tyr Ser Lys Leu Thr Val Asp Lys Ser Arg Trp
                   325                 330                 335
   Gln Gln Gly Asn Val Phe Ser Cys Ser Val Met His Glu Ala Leu His
               340                 345                 350
   Asn His Tyr Thr Gln Lys Ser Leu Ser Leu Ser Pro Gly Lys
           355                 360                 365
<210> 7
   <211> 1005
   <212> DNA
   <213> Homo sapiens
<220>
   <221> CDS
   <222> (1)..(1005)
   <223>
<400> 7
   atg gag aca gac aca ctc ctg tta tgg gtg ctg ctg ctc tgg gtt cca        48
   Met Glu Thr Asp Thr Leu Leu Leu Trp Val Leu Leu Leu Trp Val Pro
   1               5                   10                  15
   ggt tcc act ggt gac gtc aga tcc tgc ccc gaa gag cag tac tgg gat        96
   Gly Ser Thr Gly Asp Val Arg Ser Cys Pro Glu Glu Gln Tyr Trp Asp
               20                  25                  30
   cct ctg ctg ggt acc tgc atg tcc tgc aaa acc att tgc aac cat cag       144
   Pro Leu Leu Gly Thr Cys Met Ser Cys Lys Thr Ile Cys Asn His Gln
           35                  40                  45
   agc cag cgc acc tgt gca gcc ttc tgc agg tca ctc agc tgc cgc aag       192
   Ser Gln Arg Thr Cys Ala Ala Phe Cys Arg Ser Leu Ser Cys Arg Lys
       50                  55                  60
   gag caa ggc aag ttc tat gac cat ctc ctg agg gac tgc atc agc tgt       240
   Glu Gln Gly Lys Phe Tyr Asp His Leu Leu Arg Asp Cys Ile Ser Cys
   65                  70                  75                  80
   gcc tcc atc tgt gga cag cac cct aag caa tgt gca tac ttc tgt gag       288
   Ala Ser Ile Cys Gly Gln His Pro Lys Gln Cys Ala Tyr Phe Cys Glu
                   85                  90                  95
   aac aag ctc agg agc cca gtg aac ctt cca gtc gac aaa act cac aca       336
   Asn Lys Leu Arg Ser Pro Val Asn Leu Pro Val Asp Lys Thr His Thr
               100                 105                 110
   tgc cca ccg tgc cca gca cct gaa ctc ctg ggg gga ccg tca gtc ttc       384
   Cys Pro Pro Cys Pro Ala Pro Glu Leu Leu Gly Gly Pro Ser Val Phe
           115                 120                 125
   ctc ttc ccc cca aaa ccc aag gac acc ctc atg atc tcc cgg acc cct       432
   Leu Phe Pro Pro Lys Pro Lys Asp Thr Leu Met Ile Ser Arg Thr Pro
       130                 135                 140
   gag gtc aca tgc gtg gtg gtg gac gtg agc cac gaa gac cct gag gtc       480
   Glu Val Thr Cys Val Val Val Asp Val Ser His Glu Asp Pro Glu Val
   145                 150                 155                 160
   aag ttc aac tgg tac gtg gac ggc gtg gag gtg cat aat gcc aag aca       528
   Lys Phe Asn Trp Tyr Val Asp Gly Val Glu Val His Asn Ala Lys Thr
                   165                 170                 175
   aag ccg cgg gag gag cag tac aac agc acg tac cgt gtg gtc agc gtc       576
   Lys Pro Arg Glu Glu Gln Tyr Asn Ser Thr Tyr Arg Val Val Ser Val
               180                 185                 190
   ctc acc gtc ctg cac cag gac tgg ctg aat ggc aag gag tac aag tgc       624
   Leu Thr Val Leu His Gln Asp Trp Leu Asn Gly Lys Glu Tyr Lys Cys
           195                 200                 205
   aag gtc tcc aac aaa gcc ctc cca gcc ccc atc gag aaa acc atc tcc       672
   Lys Val Ser Asn Lys Ala Leu Pro Ala Pro Ile Glu Lys Thr Ile Ser
       210                 215                 220
   aaa gcc aaa ggg cag ccc cga gaa cca cag gtg tac acc ctg ccc cca      720
   Lys Ala Lys Gly Gln Pro Arg Glu Pro Gln Val Tyr Thr Leu Pro Pro
   225                 230                 235                 240
   tcc cgg gat gag ctg acc aag aac cag gtc agc ctg acc tgc ctg gtc      768
   Ser Arg Asp Glu Leu Thr Lys Asn Gln Val Ser Leu Thr Cys Leu Val
                   245                 250                 255
   aaa ggc ttc tat ccc agc gac atc gcc gtg gag tgg gag agc aat ggg      816
   Lys Gly Phe Tyr Pro Ser Asp Ile Ala Val Glu Trp Glu Ser Asn Gly
               260                 265                 270
   cag ccg gag aac aac tac aag acc acg cct ccc gtg ttg gac tcc gac      864
   Gln Pro Glu Asn Asn Tyr Lys Thr Thr Pro Pro Val Leu Asp Ser Asp
           275                 280                 285
   ggc tcc ttc ttc ctc tac agc aag ctc acc gtg gac aag agc agg tgg      912
   Gly Ser Phe Phe Leu Tyr Ser Lys Leu Thr Val Asp Lys Ser Arg Trp
       290                 295                 300
   cag cag ggg aac gtc ttc tca tgc tcc gtg atg cat gag gct ctg cac      960
   Gln Gln Gly Asn Val Phe Ser Cys Ser Val Met His Glu Ala Leu His
   305                 310                 315                 320
   aac cac tac acg cag aag agc ctc tcc ctg tct ccc ggg aaa tga         1005
   Asn His Tyr Thr Gln Lys Ser Leu Ser Leu Ser Pro Gly Lys
                   325                 330
<210> 8
   <211> 334
   <212> PRT
   <213> Homo sapiens
<400> 8
   Met Glu Thr Asp Thr Leu Leu Leu Trp Val Leu Leu Leu Trp Val Pro
   1               5                   10                  15
   Gly Ser Thr Gly Asp Val Arg Ser Cys Pro Glu Glu Gln Tyr Trp Asp
               20                  25                  30
   Pro Leu Leu Gly Thr Cys Met Ser Cys Lys Thr Ile Cys Asn His Gln
           35                  40                  45
   Ser Gln Arg Thr Cys Ala Ala Phe Cys Arg Ser Leu Ser Cys Arg Lys
       50                  55                  60
   Glu Gln Gly Lys Phe Tyr Asp His Leu Leu Arg Asp Cys Ile Ser Cys
   65                  70                  75                  80
   Ala Ser Ile Cys Gly Gln His Pro Lys Gln Cys Ala Tyr Phe Cys Glu
                   85                  90                  95
   Asn Lys Leu Arg Ser Pro Val Asn Leu Pro Val Asp Lys Thr His Thr
               100                 105                 110
   Cys Pro Pro Cys Pro Ala Pro Glu Leu Leu Gly Gly Pro Ser Val Phe
           115                 120                 125
   Leu Phe Pro Pro Lys Pro Lys Asp Thr Leu Met Ile Ser Arg Thr Pro
       130                 135                 140
   Glu Val Thr Cys Val Val Val Asp Val Ser His Glu Asp Pro Glu Val
   145                 150                 155                     160
   Lys Phe Asn Trp Tyr Val Asp Gly Val Glu Val His Asn Ala Lys Thr
                   165                 170                 175
   Lys Pro Arg Glu Glu Gln Tyr Asn Ser Thr Tyr Arg Val Val Ser Val
               180                 185                 190
   Leu Thr Val Leu His Gln Asp Trp Leu Asn Gly Lys Glu Tyr Lys Cys
           195                 200                 205
   Lys Val Ser Asn Lys Ala Leu Pro Ala Pro Ile Glu Lys Thr Ile Ser
       210                 215                 220
   Lys Ala Lys Gly Gln Pro Arg Glu Pro Gln Val Tyr Thr Leu Pro Pro
   225                 230                 235                     240
   Ser Arg Asp Glu Leu Thr Lys Asn Gln Val Ser Leu Thr Cys Leu Val
                   245                 250                 255
   Lys Gly Phe Tyr Pro Ser Asp Ile Ala Val Glu Trp Glu Ser Asn Gly
               260                 265                 270
   Gln Pro Glu Asn Asn Tyr Lys Thr Thr Pro Pro Val Leu Asp Ser Asp
           275                 280                 285
   Gly Ser Phe Phe Leu Tyr Ser Lys Leu Thr Val Asp Lys Ser Arg Trp
       290                 295                 300
   Gln Gln Gly Asn Val Phe Ser Cys Ser Val Met His Glu Ala Leu His
   305                 310                 315                     320
   Asn His Tyr Thr Gln Lys Ser Leu Ser Leu Ser Pro Gly Lys
                   325                 330
<210> 9
   <211> 531
   <212> DNA
   <213> Homo sapiens
<220>
   <221> CDS
   <222> (1)..(531)
   <223>
<400> 9
   atg gct atc atc tac ctc atc ctc ctg ttc acc gct gtg cgg ggc gat       48
   Met Ala Ile Ile Tyr Leu Ile Leu Leu Phe Thr Ala Val Arg Gly Asp
   1               5                   10                  15
   tac aaa gac gat gac gat aaa gga ccc gga cag gtg cag ctg cag gca       96
   Tyr Lys Asp Asp Asp Asp Lys Gly Pro Gly Gln Val Gln Leu Gln Ala
               20                  25                  30
   gtg ctc acc caa aaa cag aag aag cag cac tct gtc ctg cac ctg gtt      144
   Val Leu Thr Gln Lys Gln Lys Lys Gln His Ser Val Leu His Leu Val
           35                  40                  45
   ccc att aac gcc acc tcc aag gat gac tcc gat gtg aca gag gtg atg      192
   Pro Ile Asn Ala Thr Ser Lys Asp Asp Ser Asp Val Thr Glu Val Met
       50                  55                  60
   tgg caa cca gct ctt agg cgt ggg aga ggc cta cag gcc caa gga tat      240
   Trp Gln Pro Ala Leu Arg Arg Gly Arg Gly Leu Gln Ala Gln Gly Tyr
   65                  70                  75                  80
   ggt gtc cga atc cag gat gct gga gtt tat ctg ctg tat agc cag gtc      288
   Gly Val Arg Ile Gln Asp Ala Gly Val Tyr Leu Leu Tyr Ser Gln Val
                   85                  90                  95
   ctg ttt caa gac gtg act ttc acc atg ggt cag gtg gtg tct cga gaa      336
   Leu Phe Gln Asp Val Thr Phe Thr Met Gly Gln Val Val Ser Arg Glu
               100                 105                 110
   ggc caa gga agg cag gag act cta ttc cga tgt ata aga agt atg ccc      384
   Gly Gln Gly Arg Gln Glu Thr Leu Phe Arg Cys Ile Arg Ser Met Pro
           115                 120                 125
   tcc cac ccg gac cgg gcc tac aac agc tgc tat agc gca ggt gtc ttc      432
   Ser His Pro Asp Arg Ala Tyr Asn Ser Cys Tyr Ser Ala Gly Val Phe
       130                 135                 140
   cat tta cac caa ggg gat att ctg agt gtc ata att ccc cgg gca agg      480
   His Leu His Gln Gly Asp Ile Leu Ser Val Ile Ile Pro Arg Ala Arg
   145                 150                 155                 160
   gcg aaa ctt aac ctc tct cca cat gga acc ttc ctg ggg ttt gtg aaa      528
   Ala Lys Leu Asn Leu Ser Pro His Gly Thr Phe Leu Gly Phe Val Lys
                   165                 170                 175
   ctg                                                                  531
   Leu
<210> 10
   <211> 177
   <212> PRT
   <213> Homo sapiens
<400> 10
   Met Ala Ile Ile Tyr Leu Ile Leu Leu Phe Thr Ala Val Arg Gly Asp
   1               5                   10                  15
   Tyr Lys Asp Asp Asp Asp Lys Gly Pro Gly Gln Val Gln Leu Gln Ala
               20                  25                  30
   Val Leu Thr Gln Lys Gln Lys Lys Gln His Ser Val Leu His Leu Val
           35                  40                  45
   Pro Ile Asn Ala Thr Ser Lys Asp Asp Ser Asp Val Thr Glu Val Met
       50                  55                  60
   Trp Gln Pro Ala Leu Arg Arg Gly Arg Gly Leu Gln Ala Gln Gly Tyr
   65                  70                  75                  80
   Gly Val Arg Ile Gln Asp Ala Gly Val Tyr Leu Leu Tyr Ser Gln Val
                   85                  90                  95
   Leu Phe Gln Asp Val Thr Phe Thr Met Gly Gln Val Val Ser Arg Glu
               100                 105                 110
   Gly Gln Gly Arg Gln Glu Thr Leu Phe Arg Cys Ile Arg Ser Met Pro
           115                 120                 125
   Ser His Pro Asp Arg Ala Tyr Asn Ser Cys Tyr Ser Ala Gly Val Phe
       130                 135                 140
   His Leu His Gln Gly Asp Ile Leu Ser Val Ile Ile Pro Arg Ala Arg
   145                 150                 155                 160
   Ala Lys Leu Asn Leu Ser Pro His Gly Thr Phe Leu Gly Phe Val Lys
                   165                 170                 175
   Leu
<210> 11
   <211> 1470
   <212> DNA
   <213> Homo sapiens
<220>
   <221> CDS
   <222> (119)..(1021)
   <223>
<220>
   <221> CDS
   <222> (1025)..(1054)
   <223>
<400> 11
   acgcaaatgg gcggtaggcg tgtacggtgg gaggtctata taagcagagc tcgtttagtg      60
   aaccgtcaga tctctagaag ctgggtacca gctgctagca agcttgctag cggccgcc       118
   atg gac tac aaa gac gat gac gac aag agt ggc ctg ggc cgg agc agg       166
   Met Asp Tyr Lys Asp Asp Asp Asp Lys Ser Gly Leu Gly Arg Ser Arg
   1               5                   10                  15
   cga ggt ggc cgg agc cgt gtg gac cag gag gag cgc ttt cca cag ggc       214
   Arg Gly Gly Arg Ser Arg Val Asp Gln Glu Glu Arg Phe Pro Gln Gly
               20                  25                  30
   ctg tgg acg ggg gtg gct atg aga tcc tgc ccc gaa gag cag tac tgg       262
   Leu Trp Thr Gly Val Ala Met Arg Ser Cys Pro Glu Glu Gln Tyr Trp
           35                  40                  45
   gat cct ctg ctg ggt acc tgc atg tcc tgc aaa acc att tgc aac cat       310
   Asp Pro Leu Leu Gly Thr Cys Met Ser Cys Lys Thr Ile Cys Asn His
       50                  55                  60
   cag agc cag cgc acc tgt gca gcc ttc tgc agg tca ctc agc tgc cgc       358
   Gln Ser Gln Arg Thr Cys Ala Ala Phe Cys Arg Ser Leu Ser Cys Arg
   65                  70                  75                  80
   aag gag caa ggc aag ttc tat gac cat ctc ctg agg gac tgc atc agc       406
   Lys Glu Gln Gly Lys Phe Tyr Asp His Leu Leu Arg Asp Cys Ile Ser
                   85                  90                  95
   tgt gcc tcc atc tgt gga cag cac cct aag caa tgt gca tac ttc tgt       454
   Cys Ala Ser Ile Cys Gly Gln His Pro Lys Gln Cys Ala Tyr Phe Cys
               100                 105                 110
   gag aac aag ctc agg agc cca gtg aac ctt cca cca gag ctc agg aga      502
   Glu Asn Lys Leu Arg Ser Pro Val Asn Leu Pro Pro Glu Leu Arg Arg
           115                 120                 125
   cag cgg agt gga gaa gtt gaa aac aat tca gac aac tcg gga agg tac      550
   Gln Arg Ser Gly Glu Val Glu Asn Asn Ser Asp Asn Ser Gly Arg Tyr
       130                 135                 140
   caa gga ttg gag cac aga ggc tca gaa gca agt cca gct ctc ccg ggg      598
   Gln Gly Leu Glu His Arg Gly Ser Glu Ala Ser Pro Ala Leu Pro Gly
   145                 150                 155                 160
   ctg aag ctg agt gca gat cag gtg gcc ctg gtc tac agc acg ctg ggg      646
   Leu Lys Leu Ser Ala Asp Gln Val Ala Leu Val Tyr Ser Thr Leu Gly
                   165                 170                 175
   ctc tgc ctg tgt gcc gtc ctc tgc tgc ttc ctg gtg gcg gtg gcc tgc      694
   Leu Cys Leu Cys Ala Val Leu Cys Cys Phe Leu Val Ala Val Ala Cys
               180                 185                 190
   ttc ctc aag aag agg ggg gat ccc tgc tcc tgc cag ccc cgc tca agg      742
   Phe Leu Lys Lys Arg Gly Asp Pro Cys Ser Cys Gln Pro Arg Ser Arg
           195                 200                 205
   ccc cgt caa agt ccg gcc aag tct tcc cag gat cac gcg atg gaa gcc      790
   Pro Arg Gln Ser Pro Ala Lys Ser Ser Gln Asp His Ala Met Glu Ala
       210                 215                 220
   ggc agc cct gtg agc aca tcc ccc gag cca gtg gag acc tgc agc ttc      838
   Gly Ser Pro Val Ser Thr Ser Pro Glu Pro Val Glu Thr Cys Ser Phe
   225                 230                 235                 240
   tgc ttc cct gag tgc agg gcg ccc acg cag gag agc gca gtc acg cct      886
   Cys Phe Pro Glu Cys Arg Ala Pro Thr Gln Glu Ser Ala Val Thr Pro
                   245                 250                 255
   ggg acc ccc gac ccc act tgt gct gga agg tgg ggg tgc cac acc agg      934
   Gly Thr Pro Asp Pro Thr Cys Ala Gly Arg Trp Gly Cys His Thr Arg
               260                 265                 270
   acc aca gtc ctg cag cct tgc cca cac atc cca gac agt ggc ctt ggc      982
   Thr Thr Val Leu Gln Pro Cys Pro His Ile Pro Asp Ser Gly Leu Gly
           275                 280                 285
   att gtg tgt gtg cct gcc cag gag ggg ggc cca ggt gca taa atg ggg     1030
   Ile Val Cys Val Pro Ala Gln Glu Gly Gly Pro Gly Ala     Met Gly
       290                 295                 300
   gtc agc ggc cgc tcg agg ccg gca aggccggatc cagacatgat aagatacatt    1084
   Val Ser Gly Arg Ser Arg Pro Ala
       305                 310
   gatgagtttg gacaaaccac aactagaatg cagtgaaaaa aatgctttat ttgtgaaatt   1144
   tgtgatgcta ttgctttatt tgtaaccatt ataagctgca ataaacaagt taacaacaac   1204
   aattgcattc attttatgtt tcaggttcag ggggaggtgg ggaggttttt taaagcaagt 1264
   aaaacctcta caaatgtggt atggctgatt atgatccggc tgcctcgcgc gtttcggtga 1324
   tgacggtgaa aacctctgac acatgcagct cccggagacg gtcacagctt gtctgtaagc 1384
   ggatgccggg agcagacaag cccgtcaggg cgcgtcagcg ggtgttggcg ggtgtcgggg 1444
   cgcagccatg accggtcgac tctaga 1470
<210> 12
   <211> 301
   <212> PRT
   <213> Homo sapiens
<400> 12
   Met Asp Tyr Lys Asp Asp Asp Asp Lys Ser Gly Leu Gly Arg Ser Arg
   1               5                   10                  15
   Arg Gly Gly Arg Ser Arg Val Asp Gln Glu Glu Arg Phe Pro Gln Gly
               20                  25                  30
   Leu Trp Thr Gly Val Ala Met Arg Ser Cys Pro Glu Glu Gln Tyr Trp
           35                  40                  45
   Asp Pro Leu Leu Gly Thr Cys Met Ser Cys Lys Thr Ile Cys Asn His
       50                  55                  60
   Gln Ser Gln Arg Thr Cys Ala Ala Phe Cys Arg Ser Leu Ser Cys Arg
   65                  70                  75                  80
   Lys Glu Gln Gly Lys Phe Tyr Asp His Leu Leu Arg Asp Cys Ile Ser
                   85                  90                  95
   Cys Ala Ser Ile Cys Gly Gln His Pro Lys Gln Cys Ala Tyr Phe Cys
               100                 105                 110
   Glu Asn Lys Leu Arg Ser Pro Val Asn Leu Pro Pro Glu Leu Arg Arg
           115                 120                 125
   Gln Arg Ser Gly Glu Val Glu Asn Asn Ser Asp Asn Ser Gly Arg Tyr
       130                 135                 140
   Gln Gly Leu Glu His Arg Gly Ser Glu Ala Ser Pro Ala Leu Pro Gly
   145                 150                 155                 160
   Leu Lys Leu Ser Ala Asp Gln Val Ala Leu Val Tyr Ser Thr Leu Gly
                   165                 170                 175
   Leu Cys Leu Cys Ala Val Leu Cys Cys Phe Leu Val Ala Val Ala Cys
               180                 185                 190
   Phe Leu Lys Lys Arg Gly Asp Pro Cys Ser Cys Gln Pro Arg Ser Arg
           195                 200                 205
   Pro Arg Gln Ser Pro Ala Lys Ser Ser Gln Asp His Ala Met Glu Ala
       210                 215                 220
   Gly Ser Pro Val Ser Thr Ser Pro Glu Pro Val Glu Thr Cys Ser Phe
   225                 230                 235                 240
   Cys Phe Pro Glu Cys Arg Ala Pro Thr Gln Glu Ser Ala Val Thr Pro
                   245                 250                 255
   Gly Thr Pro Asp Pro Thr Cys Ala Gly Arg Trp Gly Cys His Thr Arg
               260                 265                 270
   Thr Thr Val Leu Gln Pro Cys Pro His Ile Pro Asp Ser Gly Leu Gly
           275                 280                 285
   Ile Val Cys Val Pro Ala Gln Glu Gly Gly Pro Gly Ala
       290                 295                 300
<210> 13
   <211> 10
   <212> PRT
   <213> Homo sapiens
<400> 13
   Met Gly Val Ser Gly Arg Ser Arg Pro Ala
   1               5                   10
<210> 14
   <211> 1304
   <212> DNA
   <213> Homo sapiens
<220>
   <221> CDS
   <222> (42)..(1253)
   <223>
<400> 14
   taatacgact cactataggg agacccaagc ttaatcaaaa c atg gct atc atc tac      56
   ctc atc ctc ctg ttc acc gct gtg cgg ggc ctc gac atg agt ggc ctg       104
   ggc cgg agc agg cga ggt ggc cgg agc cgt gtg gac cag gag gag cgc       152
   ttt cca cag ggc ctg tgg acg ggg gtg gct atg aga tcc tgc ccc gaa       200
   gag cag tac tgg gat cct ctg ctg ggt acc tgc atg tcc tgc aaa acc       248
   att tgc aac cat cag agc cag cgc acc tgt gca gcc ttc tgc agg tca       296
   ctc agc tgc cgc aag gag caa ggc aag ttc tat gac cat ctc ctg agg       344
   gac tgc atc agc tgt gcc tcc atc tgt gga cag cac cct aag caa tgt       392
   gca tac ttc tgt gag aac aag ctc agg agc cca gtg aac ctt cca cca       440
   gag ctc agg aga cag cgg agt gga gaa gtt gaa aac aat tca gac aac       488
   tcg gga agg tac caa gga ttg gag cac aga ggc tca gaa gca agt cca       536
   gct ctc ccg ggg ctg aag ctg agt gca gat cag gtc gac aaa act cac       584
   aca tgc cca ccg tgc cca gca cct gaa ctc ctg ggg gga ccg tca gtc       632
   ttc ctc ttc ccc cca aaa ccc aag gac acc ctc atg atc tcc cgg acc       680
   cct gag gtc aca tgc gtg gtg gtg gac gtg agc cac gaa gac cct gag        728
   gtc aag ttc aac tgg tac gtg gac ggc gtg gag gtg cat aat gcc aag        776
   aca aag ccg cgg gag gag cag tac aac agc acg tac cgt gtg gtc agc        824
   gtc ctc acc gtc ctg cac cag gac tgg ctg aat ggc aag gag tac aag        872
   tgc aag gtc tcc aac aaa gcc ctc cca gcc ccc atc gag aaa acc atc        920
   tcc aaa gcc aaa ggg cag ccc cga gaa cca cag gtg tac acc ctg ccc        968
   cca tcc cgg gat gag ctg acc aag aac cag gtc agc ctg acc tgc ctg       1016
   gtc aaa ggc ttc tat ccc agc gac atc gcc gtg gag tgg gag agc aat       1064
   ggg cag ccg gag aac aac tac aag acc acg cct ccc gtg ttg gac tcc       1112
   gac ggc tcc ttc ttc ctc tac agc aag ctc acc gtg gac aag agc agg       1160
   tgg cag cag ggg aac gtc ttc tca tgc tcc gtg atg cat gag gct ctg       1208
   cac aac cac tac acg cag aag agc ctc tcc ctg tct ccg ggt aaa           1253
   tgagtgcgcg cggccgctct agagggccct attctatagt gtcacctaaa t              1304
<210> 15
   <211> 404
   <212> PRT
   <213> Homo sapiens
<400> 15
<210> 16
   <211> 546
   <212> DNA
   <213> Mus musculus
<220>
   <221> CDS
   <222> (16)..(543)
   <223>
<400> 16
   aagcttaatc aaaac atg gct atc atc tac ctc atc ctc ctg ttc acc gct       51
                    Met Ala Ile Ile Tyr Leu Ile Leu Leu Phe Thr Ala
                    1               5                   10
   gtg cgg ggc gat tac aaa gac gat gac gat aaa gga ccc gga cag gtg        99
   Val Arg Gly Asp Tyr Lys Asp Asp Asp Asp Lys Gly Pro Gly Gln Val
           15                  20                  25
   cag ctg cag gca gta ctc acc cag aag cac aag aag aag cac tca gtc       147
   Gln Leu Gln Ala Val Leu Thr Gln Lys His Lys Lys Lys His Ser Val
       30                  35                  40
   ctg cat ctt gtt cca gtt aac att acc tcc aag gac tct gac gtg aca       195
   Leu His Leu Val Pro Val Asn Ile Thr Ser Lys Asp Ser Asp Val Thr
   45                  50                  55                  60
   gag gtg atg tgg caa cca gta ctt agg cgt ggg aga ggc ctg gag gcc       243
   Glu Val Met Trp Gln Pro Val Leu Arg Arg Gly Arg Gly Leu Glu Ala
                   65                  70                  75
   cag gga gac att gta cga gtc tgg gac act gga att tat ctg ctc tat       291
   Gln Gly Asp Ile Val Arg Val Trp Asp Thr Gly Ile Tyr Leu Leu Tyr
               80                  85                  90
   agt cag gtc ctg ttt cat gat gtg act ttc aca atg ggt cag gtg gta       339
   Ser Gln Val Leu Phe His Asp Val Thr Phe Thr Met Gly Gln Val Val
           95                  100                 105
   tct cgg gaa gga caa ggg aga aga gaa act cta ttc cga tgt atc aga       387
   Ser Arg Glu Gly Gln Gly Arg Arg Glu Thr Leu Phe Arg Cys Ile Arg
       110                 115                 120
   agt atg cct tct gat cct gac cgt gcc tac aat agc tgc tac agt gca       435
   Ser Met Pro Ser Asp Pro Asp Arg Ala Tyr Asn Ser Cys Tyr Ser Ala
   125                 130                 135                 140
   ggt gtc ttt cat tta cat caa ggg gat att atc act gtc aaa att cca       483
   Gly Val Phe His Leu His Gln Gly Asp Ile Ile Thr Val Lys Ile Pro
                   145                 150                 155
   cgg gca aac gca aaa ctt agc ctt tct ccg cat gga aca ttc ctg ggg       531
   Arg Ala Asn Ala Lys Leu Ser Leu Ser Pro His Gly Thr Phe Leu Gly
               160                 165                 170
   ttt gtg aaa cta tga                                                   546
   Phe Val Lys Leu
           175
<210> 17
   <211> 176
   <212> PRT
   <213> Mus musculus
<400> 17
   Met Ala Ile Ile Tyr Leu Ile Leu Leu Phe Thr Ala Val Arg Gly Asp
   1               5                   10                  15
   Tyr Lys Asp Asp Asp Asp Lys Gly Pro Gly Gln Val Gln Leu Gln Ala
               20                  25                  30
   Val Leu Thr Gln Lys His Lys Lys Lys His Ser Val Leu His Leu Val
           35                  40                  45
   Pro Val Asn Ile Thr Ser Lys Asp Ser Asp Val Thr Glu Val Met Trp
       50                  55                  60
   Gln Pro Val Leu Arg Arg Gly Arg Gly Leu Glu Ala Gln Gly Asp Ile
   65                  70                  75                      80
   Val Arg Val Trp Asp Thr Gly Ile Tyr Leu Leu Tyr Ser Gln Val Leu
                   85                  90                  95
   Phe His Asp Val Thr Phe Thr Met Gly Gln Val Val Ser Arg Glu Gly
               100                 105                 110
   Gln Gly Arg Arg Glu Thr Leu Phe Arg Cys Ile Arg Ser Met Pro Ser
           115                 120                 125
   Asp Pro Asp Arg Ala Tyr Asn Ser Cys Tyr Ser Ala Gly Val Phe His
       130                 135                 140
   Leu His Gln Gly Asp Ile Ile Thr Val Lys Ile Pro Arg Ala Asn Ala
   145                 150                 155                     160
   Lys Leu Ser Leu Ser Pro His Gly Thr Phe Leu Gly Phe Val Lys Leu
                   165                 170                 175